# EUROPEAN PATENT SPECIFICATION

(11) **EP 3 895 674 B1**
(45) Date of publication and mention of the grant of the patent: **05.07.2023**
(21) Application number: 21178004.4
(22) Date of filing: 07.06.2021
(51) Int. Cl.: A61F 13/15

(54) **METHOD FOR MANUFACTURING ABSORBENT ARTICLE**
VERFAHREN ZUR HERSTELLUNG EINES ABSORBIERENDEN GEGENSTANDS
METHODE DE FABRICATION D'UN ARTICLE ABSORBANT

(43) Date of publication of application: 20.10.2021
(73) Proprietor: Unicharm Corporation, Shikokuchuo-shi, Ehime 799-0111 (JP)
(72) Inventor: ITO, Yoshihiro, Kanonji-shi, 769-1602 (JP); FUJIMOTO, Kazuya, Kanonji-shi, 769-1602 (JP); UDAKA, Hikari, Kanonji-shi, 769-1602 (JP)
(74) Representative: Dolleymores

(56) References cited:
- EP-B1- 0 680 301
- WO-A1-2016/194475
- WO-A1-2016/194476
- WO-A1-2016/194477
- WO-A1-2016/194480

## Description

### [Technical Field]

The present invention relates to a method for manufacturing an absorbent article.

### [Background Art]

There are already known methods for manufacturing an absorbent article by overlaying a stretchy member, which has leg openings and a waist opening, on an absorbent main body and then joining the absorbent main body and the stretchy member to manufacture an absorbent article. Examples of such absorbent articles include pull-on disposable diapers and the like.

### [Citation List]

### [Patent Literature]

[Patent Document 1] Japanese Unexamined Patent Application Publication (Translation of PCT Application) No. H08-507699
[Patent Document 2] Japanese Patent Application Publication No. H09-010261

WO 2016/194475 A1, WO 2016/194476 A1 and WO 2016/194477 A1 all relate to a manufacturing method for an absorbent article. The absorbent article comprises, in an incomplete state, an absorbent body that absorbs liquids, and a pair of belt members that are arranged side-by-side in the width direction, which is orthogonal to the longitudinal direction of the absorbent body, while overlapping with the absorbent body.

### [Summary of Invention]

### [Technical Problem]

When manufacturing such an absorbent article, in order to improve the functionality of the stretchy member that has the leg openings and the waist opening, an auxiliary member such as a stretchable member or a reinforcing member is sometimes arranged around the leg and/or waist openings. Conventionally, when such an auxiliary member is arranged on a pair of stretchy members that have leg and waist openings at the time of manufacturing, there are cases where two rolls of the wound auxiliary member are prepared, and an auxiliary-member continuous body is fed out from each of the rolls. However, if the auxiliary member is a stretchable member or the like, the auxiliary member wound in the roll sometimes has a certain amount of variation in width in the first place, and the widthwise end portions of the auxiliary member are sometimes not stable to due to deformation or misalignment. If the step of finally forming joining portions, in which the auxiliary member is joined together with the absorbent main body, is performed in such a state, there is a risk that there will be different numbers of overlaid materials in the range where the joining portions are formed, and that products whose joining portions have different joining strengths will be formed.

Also, in the foregoing case where two material rolls of auxiliary members are prepared for the step of arranging the auxiliary members, it is necessary to also provide two material feeding devices and joining devices, as well as devices for controlling the tensions thereof, for example. This is more likely to cause problems such as the need for excessive equipment space and complicated control.

The present invention has been made in view of the foregoing problems, and an object of the present invention is to reduce width-direction variation in the arrangement position of an auxiliary member that is to be arranged on a composite member that has leg openings and a waist opening, stabilizing the joining strength of a joining portion in which the auxiliary member is joined to an absorbent main body.

### [Solution to Problem]

A main aspect of the present invention for achieving the above-described aspect is a method for manufacturing an absorbent article, the absorbent article including an absorbent main body, a pair of composite members, and an auxiliary member, the absorbent main body absorbing a liquid, the pair of composite members having leg openings and a waist opening, the auxiliary member being provided on each of the composite members, the method including: a feeding step of feeding an auxiliary-member continuous body from a roll, the auxiliary-member continuous body being for forming the auxiliary member, the auxiliary-member continuous body being wound around the roll; a first joining step of joining a composite-member continuous body and the auxiliary-member continuous body that has been fed from the roll, the composite-member continuous body being for forming the composite member; a second joining step of joining an absorbent-main-body continuous body to the auxiliary-member continuous body and the composite-member continuous body that have been joined in the first joining step, the second joining step being performed by forming a joining portion, the joining portion intersecting a direction of transport, the absorbent-main-body continuous body being for forming the absorbent main body; and a dividing step of dividing the auxiliary-member continuous body at a predetermined position in an intersecting direction, the dividing step being performed between the feeding step and the second joining step, the intersecting direction being a direction that intersects the direction of transport.

Features of the present invention other than the above will become clear by reading the description of the present specification with reference to the accompanying drawings.

### [Advantageous Effects of Invention]

According to the present invention, it is possible to reduce width-direction variation in the arrangement position of an auxiliary member that is to be arranged on a composite member that has leg openings and a waist opening, stabilizing the joining strength of a joining portion in which the auxiliary member is joined to an absorbent main body.

### [Brief Description of the Drawings]

FIG. 1 is a schematic front view of a diaper 1 manufactured by a manufacturing method according to a first embodiment, as viewed from the front side.
FIG. 2 is a schematic plan view of the diaper 1 in a flat extended state before completion, as viewed from a composite member 30 side.
FIG. 3 is a schematic plan view of the diaper 1 as viewed from an absorbent main body 10 side.
FIG. 4 is a cross-sectional view taken along a line A-A in FIG. 2.
FIG. 5A is a schematic plan view of the diaper 1 in the flat extended state in FIG. 2, whose portions have been peeled and unfolded such that the skin-side surface can be seen.
FIG. 5B is a cross-sectional view taken along a line B-B in FIG. 5A.
FIG. 6 is a flowchart of steps performed when manufacturing the diaper 1 according to the first embodiment.
FIG. 7 is a schematic view showing a manufacturing line of the diaper 1 according to the first embodiment.
FIG. 8 is an illustrative diagram of a dividing step and a positioning step of the manufacturing method according to the first embodiment.
FIG. 9 is an illustrative diagram of a first joining step and a composite-member dividing step.
FIG. 10 is an illustrative diagram of a composite-member positioning step and an absorbent-main-body attaching step.
FIG. 11 is an illustrative diagram of a leg-opening forming step and a folding step.
FIG. 12 is an illustrative diagram of a second joining step and a segmenting step.
FIG. 13 is an illustrative diagram of a positioning step and a first joining step of a manufacturing method according to a second embodiment.
FIG. 14 is an illustrative diagram of an absorbent-main-body attaching step and a second joining step.
FIG. 15 is an illustrative diagram of a cutting step and a segmenting step.
FIG. 16 is an illustrative diagram of a first joining step of a manufacturing method according to a third embodiment.
FIG. 17 is an illustrative diagram of a composite-member dividing step and a composite-member positioning step.
FIG. 18 is a schematic plan view of the diaper 1 in the flat extended state in a variation, whose portions have been peeled and unfolded such that the skin-side surface can be seen.
FIG. 19 is a diagram showing a portion of a cross-sectional view taken along a line B-B in FIG. 18.

### [Description of Embodiments]

At least the following matters will become clear with the description of this specification and the attached drawings.

A method for manufacturing an absorbent article, the absorbent article including an absorbent main body, a pair of composite members, and an auxiliary member, the absorbent main body absorbing a liquid, the pair of composite members having leg openings and a waist opening, the auxiliary member being provided on each of the composite members, the method including: a feeding step of feeding an auxiliary-member continuous body from a roll, the auxiliary-member continuous body being for forming the auxiliary member, the auxiliary-member continuous body being wound around the roll; a first joining step of joining a composite-member continuous body and the auxiliary-member continuous body that has been fed from the roll, the composite-member continuous body being for forming the composite member; a second joining step of joining an absorbent-main-body continuous body to the auxiliary-member continuous body and the composite-member continuous body that have been joined in the first joining step, the second joining step being performed by forming a joining portion, the joining portion intersecting a direction of transport, the absorbent-main-body continuous body being for forming the absorbent main body; and a dividing step of dividing the auxiliary-member continuous body at a predetermined position in an intersecting direction, the dividing step being performed between the feeding step and the second joining step, the intersecting direction being a direction that intersects the direction of transport.

According to this method for manufacturing an absorbent article, there are cases where the width of the auxiliary member varies slightly, or cases where the widthwise end portions are not be stable due to deformation, misalignment, or the like. But such variation can be reduced by dividing the one auxiliary member at a predetermined position in the width direction. Accordingly, in the second joining step in which the auxiliary member is finally joined together with the composite member and the absorbent main body, the arrangement position of the auxiliary member with respect to the range of the formed joining portion is stable, making the joining strength of the joining portion more likely to be stable. Also, because the auxiliary member can be supplied by one sheet (continuous body), it is possible to reduce the amount of needed equipment space and reduce the complexity of device control.

In such a method for manufacturing an absorbent article, it is desirable that the auxiliary member is a stretchable member.

According to this method for manufacturing an absorbent article, when the auxiliary member, which is a stretchable member, is arranged on each of the pair of composite members, the stretchable member, which originally can easily vary in size in the width direction, can be arranged with less variation in the width direction in the case where one sheet is divided and then arranged than in the case where the stretchable member is feed from sheets in two rolls and then arranged.

In such a method for manufacturing an absorbent article, it is desirable that in the second joining step, the auxiliary-member continuous body is arranged so as to overlap at least one of an intersecting-direction outer end portion and an intersecting-direction inner end portion of the joining portion that is to be formed, the intersecting-direction outer end portion being a end portion located closer to outside in the intersecting direction, the intersecting-direction inner end portion being a end portion located closer to inside in the intersecting direction.

According to this method for manufacturing an absorbent article, in the product state, the outer end portions of the joining portions are included in the leg openings, and the inner end portions are included in the waist opening. Accordingly, both are portions that require strength. Because there is reduced variation in width of the auxiliary-member continuous bodies that have been divided in the dividing step, even when the joining portion is formed in the second joining step, it is possible to stably arrange the auxiliary member up to the end portion of the joining portion.

In such a method for manufacturing an absorbent article, it is desirable that the method further comprises a folding step of folding the composite-member continuous body in the intersecting direction at a folding line, the folding step being performed between the first joining step and the second joining step, the folding line being set in the composite-member continuous body, that in the folding step, the auxiliary-member continuous body is folded in the intersecting direction together with the composite-member continuous body, and that in the second joining step, the joining portion is formed such that an overlapping portion is located in at least one of the intersecting-direction outer end portion and the intersecting-direction inner end portion of the joining portion, the overlapping portion being a portion where portions of the auxiliary-member continuous body are overlapped due to the folding step.

According to this method for manufacturing an absorbent article, in the folding step, if the auxiliary-member continuous body originally has variation in the width direction, there is a risk that the amount of variation in the arrangement position of the auxiliary-member continuous body further increases due to folding. But, the amount of variation in the width direction has been reduced for the auxiliary-member continuous bodies that has been divided in the dividing step, and therefore even when the auxiliary members are arranged at the end portions of the joining portions in a folded state, such arrangement can be performed with reduced variation.

In such a method for manufacturing an absorbent article, it is desirable that in the second joining step, the auxiliary-member continuous body is arranged on both an intersecting-direction outer end portion and an intersecting-direction inner end portion of the joining portion that is to be formed, the intersecting-direction outer end portion being a end portion located closer to outside in the intersecting direction, the intersecting-direction inner end portion being a end portion located closer to inside in the intersecting direction.

According to this method for manufacturing an absorbent article, it increases the number of constituent materials that are overlaid at the two intersecting-direction end portions of the joining portion, making it easier to form the joining portion more reliably at the two end portions. This therefore makes it easier to stabilize the quality of the product.

In such a method for manufacturing an absorbent article, it is desirable that the method further comprises a cutting step of cutting the auxiliary-member continuous body together with the composite-member continuous body at a cutting line, the cutting step being performed after the second joining step, the cutting line being a line that is set in the composite-member continuous body and extends in a curved shape along the direction of transport, and the auxiliary-member continuous body is arranged at a portion that is constituted by the composite-member continuous body and that is in an edge portion of each of the leg openings formed by the cutting step.

According to this method for manufacturing an absorbent article, in the stage before the second joining step, the auxiliary-member continuous body has a reduced amount of variation in the width direction and can be stably arranged. Therefore even when the leg openings are formed by cutting the composite-member continuous body together with the auxiliary-member continuous bodies in a curved shape, the auxiliary members can be arranged appropriately up to the edge portions of the leg openings.

In such a method for manufacturing an absorbent article, it is desirable that the method further comprises: a leg-opening forming step of forming the leg openings by performing cutting in a curved shape the auxiliary-member continuous body and the composite-member continuous body so as to penetrate through the auxiliary-member continuous body and the composite-member continuous body, the auxiliary-member continuous body and the composite-member continuous body having been joined in the first joining step; and a folding step of folding the composite-member continuous body together with the auxiliary-member continuous body in the intersecting direction at a folding line, the folding step being performed after the leg-opening forming step, the folding line being set in the composite-member continuous body, and that in the leg-opening forming step, the leg openings are each formed so as to straddle the folding line in the intersecting direction.

According to this method for manufacturing an absorbent article, it is possible to reduce the amount of width-direction (intersecting-direction) variation in the position of the auxiliary member arranged at the end portions of each of the curved leg openings, making it easier to arrange appropriately the auxiliary members around the entire circumference of the leg openings. Also, by forming the leg openings before the folding step, the auxiliary member can be appropriately arranged even if the shape of the leg opening is asymmetrical in the left-right direction.

In such a method for manufacturing an absorbent article, it is desirable that in the second joining step, a pair of the joining portions are formed in the absorbent-main-body continuous body, on an upstream side in the direction of transport, and a pair of the joining portions are formed in the absorbent-main-body continuous body, on a downstream side in the direction of transport, and letting the pair of joining portions on the upstream side be a pair of first joining portions, and letting the pair of joining portions on the downstream side be a pair of second joining portions, a plurality of weld portions are provided intermittently in the first joining portions and the second joining portions, the first joining portions are each provided so as to be inclined toward a center from outside in the intersecting direction, and the second joining portions are each provided so as to be inclined toward outside in the intersecting direction from the center.

According to this method for manufacturing an absorbent article, if the weld portions of the joining portions are formed in only one direction (or continuously), it causes a risk that the continuous force that acts in the one direction makes wrinkles easier to form in the continuous body for forming the absorbent article. In the first joining portions, a force intermittently acts inward in the intersecting direction (width direction), and in the second joining portions, a force intermittently acts outward in the intersecting direction. This makes it possible to apply force alternatingly to the sheets, making wrinkles less likely to form.

In such a method for manufacturing an absorbent article, it is desirable that the auxiliary member has stretchability, and an amount of elongation per unit length of the auxiliary-member continuous body in the dividing step is smaller than the amount of elongation per unit length of the auxiliary-member continuous body in the first joining step.

According to this method for manufacturing an absorbent article, when the auxiliary member is divided, variation in the size in the width direction is reduced by suppressing elongation of the auxiliary member during the dividing. If the auxiliary member having a reduced amount of variation in width is joined to the composite member in an elongated state, it is easier to stabilize the position of the auxiliary member in the width direction.

In such a method for manufacturing an absorbent article, it is desirable that concerning a transporting distance of the auxiliary-member continuous body from dividing in the dividing step to joining in the second joining step, in manufacturing, and concerning a transporting distance of the auxiliary-member continuous body from feeding by the roll in the feeding step to the dividing in the dividing step, the former transporting distance is shorter than the latter transporting distance.

According to this method for manufacturing an absorbent article, even an auxiliary member whose width can easily vary is able to be stably transported in its wide state before being divided. After the width is reduced due to division, using a shorter transporting distance and minimizing the variation in width after division makes it possible to maintain stability.

In such a method for manufacturing an absorbent article, it is desirable that the method further comprises a positioning step of performing positioning such that a central position of the auxiliary-member continuous body in the intersecting direction is at the predetermined position in the intersecting direction, the positioning step being performed before the dividing step.

According to this method for manufacturing an absorbent article, the auxiliary-member continuous body can be divided into two portions at the central position in the intersecting direction (width direction), and the variation in the width direction of the auxiliary-member continuous body can be halved.

In such a method for manufacturing an absorbent article, it is desirable that in the dividing step, the auxiliary-member continuous body is divided along a dividing line, the dividing line being a line that is continuous in the direction of transport, and the method further comprises a positioning step of positioning the divided auxiliary-member continuous bodies based on divided ends with respect to the intersecting direction, the positioning step being performed between the dividing step and the first joining step.

According to this method for manufacturing an absorbent article, the variation in the width direction is reduced in the portions that have been cut at the dividing line, and by performing transporting based on the portions that extend along the dividing line, proper positioning is likely to be achieved in the subsequent first joining step.

In such a method for manufacturing an absorbent article, it is desirable that the dividing step is performed after the first joining step.

According to this method for manufacturing an absorbent article, first the auxiliary member and the composite member are joined, and then the auxiliary member is divided. This makes it possible to arrange the auxiliary member more correctly with respect to the composite member.

In such a method for manufacturing an absorbent article, it is desirable that in the dividing step, the auxiliary-member continuous body is divided along a dividing line, the dividing line being a line that is continuous in the direction of transport, and that of the divided auxiliary-member continuous body, ends that have been divided in the intersecting direction, are arranged at ends of the leg openings.

According to this method for manufacturing an absorbent article, the auxiliary member can be arranged very nearly up to the ends of the leg openings.

In such a method for manufacturing an absorbent article, it is desirable that in the dividing step, the auxiliary-member continuous body is divided into at least four portions at a plurality of predetermined positions in the intersecting direction, and that in the first joining step, the at least four divided portions of the auxiliary-member continuous body are arranged such that, in a pair composed of the at least four divided portions, the two divided portions have an overlapping portion, and then are joined to the composite-member continuous body.

According to this method for manufacturing an absorbent article, the variation in the width direction is reduced in each of the four auxiliary-member continuous bodies that have been obtained in the dividing step. By arranging the divided auxiliary members at appropriate positions in the first joining step, it is possible to optimize the arrangement of the constituent materials of the joining portions that are formed in the subsequent second joining step, and the strength of the joining portions can be further stabilized.

### First Embodiment

A method for manufacturing an absorbent article according to a first embodiment is used in, for example, a manufacturing line for a disposable diaper 1 serving as an example of an absorbent article.

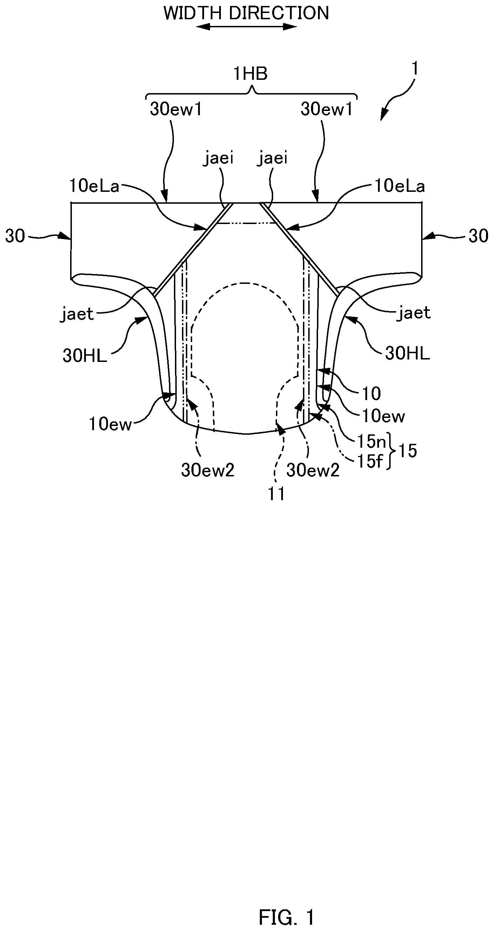

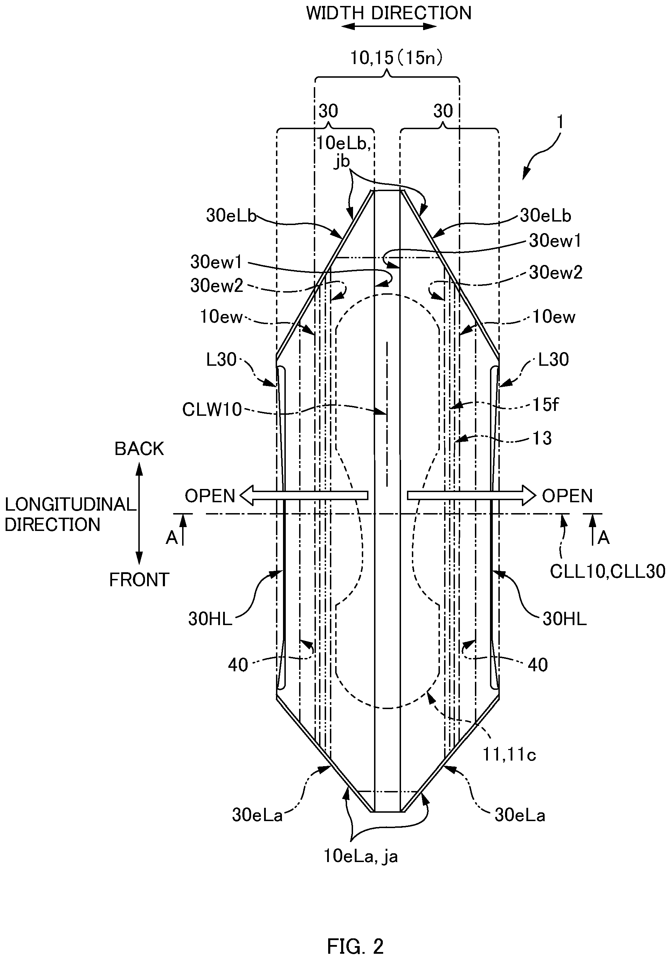

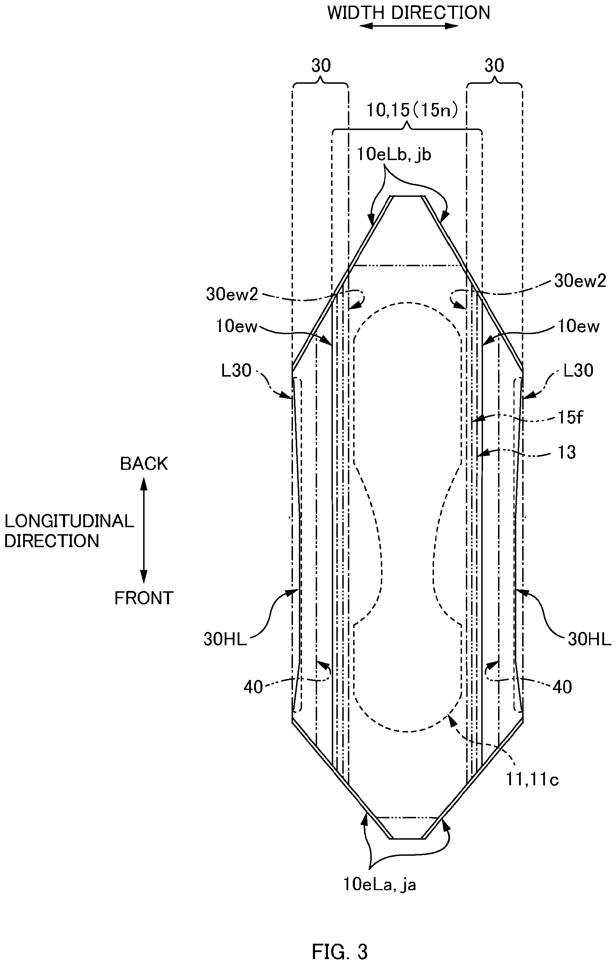

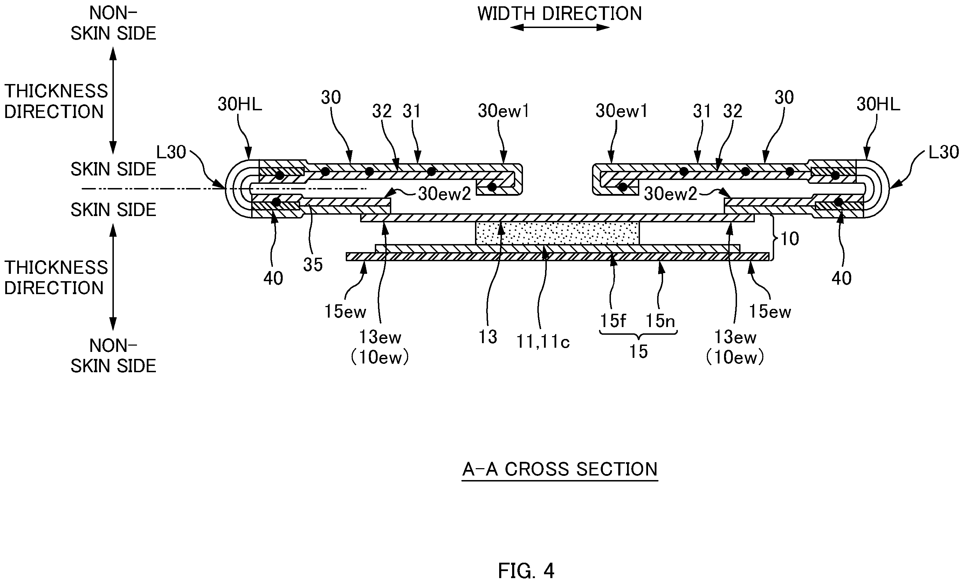

FIG. 1 is a schematic front view of an underpants-shaped diaper 1 serving as an example of the diaper 1, as viewed from the front side. FIG. 2 is a schematic plan view of the diaper 1 in a flat extended state before completion, as viewed from a composite member 30 side, and FIG. 3 is a schematic plan view of the diaper 1, as viewed from an absorbent main body 10 side. Also, FIG. 4 is a cross-sectional view taken along a line A-A in FIG. 2.

At the final stage of the manufacturing process, this diaper 1 is in a flat extended state as shown in FIG. 2. Specifically, first, the absorbent main body 10, which is to be arranged at the wearer's crotch, is a sheet-like member having a longitudinal direction, a width direction, and a thickness direction that are orthogonal to each other, and longitudinal end portions 10eLa and 10eLb of the absorbent main body 10 each have an approximately V-like tapered shape. Also, a pair of composite members 30, which are to be mainly arranged around the wearer's waist, are overlaid on the skin-side surface of the absorbent main body 10, and are arranged aligned with each other in the above-mentioned width direction; the width direction intersects the longitudinal direction of the absorbent main body 10. The composite members 30 each have a leg opening 30HL. Also, as shown in FIGS. 2 and 4, the composite members 30 respectively have folding lines L30. The folding lines L30 are for folding the composite members 30 in the width direction so that widthwise end portions 30ew1 and 30ew2 of the composite members 30 are located inside in the width direction with respect to the folding lines L30. After being folded at the folding lines L30, the composite members 30 are joined to widthwise end portions 10ew of the absorbent main body 10 by, for example, being fixed with use of a hot-melt adhesive or the like via, out of the two end portions 30ew1 and 30ew2, the end portions 30ew2 that are located outside in the width direction. Also, the composite members 30 are also joined by welding or the like in substantially V-like end portions 10eLa and 10eLb of the absorbent main body 10.

Then, in this flat extended state, the end portions 30ew1 and 30ew1 located inside of the pair of composite members 30 and 30 are spread outward in the width direction, and the composite members 30 are folded one time at an approximately central position CLL30 in the longitudinal direction. In this process, when the absorbent main body 10 is folded one time at an approximately central position CLL10 in the longitudinal direction, the widthwise inner end portions 30ew1 and 30ew1 of the composite members 30 together form a waist opening 1HB of the diaper 1. And the leg openings 30HL and 30HL of the composite members 30 become located on the two widthwise sides of the absorbent main body 10, and thus the underpants-shaped diaper 1 shown in FIG. 1 is put in a wearable state.

Note that in the following, the constituent components 10 and 30 are described mainly with reference to the diaper 1 in the flat extended state in FIG. 2, and the longitudinal direction, the width direction, and the thickness direction of the absorbent main body 10 in this flat extended state substantially coincide with the longitudinal direction, the width direction, and the thickness direction of the diaper 1 in the same flat extended state. Therefore, in the following description, these directions are simply referred to as the "longitudinal direction", the "width direction", and the "thickness direction". In the longitudinal direction, one side is located on the stomach side of the wearer and the other side is located on the back side of the wearer, and therefore the one side is also referred to as the "front side", and the other side is also referred to as the "back side". Also, in the thickness direction, the side that comes into contact with the wearer is also referred to as the "skin side", and the opposite side is also referred to as the "non-skin side". Moreover, the width direction is also referred to as the "lateral direction".

As shown in FIGS. 2 and 4, the absorbent main body 10 is a sheet-like member having longitudinal end portions 10eLa and 10eLb that are tapered in a substantially V-like shape in a plan view. The absorbent main body 10 includes: an absorbent body 11; a top sheet 13 provided so as to cover the absorbent body 11 on the skin side; and a back sheet 15 provided so as to cover the absorbent body 11 on the non-skin side.

These members 13, 11 and 15 are joined to members that are adjacent in the thickness direction with use of a hot-melt adhesive or the like. Note that examples of the application pattern of the adhesive include an Ω pattern, a spiral pattern, a stripe pattern, and the like, and this similarly applies to other adhesives that will appear later.

The absorbent body 11 has a liquid-absorbing absorbent core 11c and a core-wrapping sheet (not shown) that covers the outer peripheral surface of the core 11c. The absorbent core 11c is a molded body obtained by molding a predetermined liquid absorbent material into a substantially hourglass shape in a plan view, as one example of a predetermined shape. Examples of the liquid absorbent material include liquid-absorbent fibers (e.g., pulp fibers) and liquid-absorbent particulate matter (e.g., superabsorbent polymers, so-called SAP). Also, a liquid-permeable sheet made of tissue paper, nonwoven fabric, or the like can be used as the core-wrapping sheet. Also, the shape of the absorbent core 11c is not limited to the above-mentioned substantially hourglass shape in a plan view, and may be another shape.

The top sheet 13 is formed of a liquid-permeable sheet such as an air-through nonwoven fabric sheet, and its planar size is set such that it projects from both longitudinal sides and both widthwise sides of the absorbent body 11. Also, because the longitudinal end portions constitute the end portion 10eLa and 10eLb of the absorbent main body 10, they have the above-mentioned approximately V-shaped tapered shape of becoming smaller in the width direction toward the front side or the back side in the longitudinal direction.

Also, the back sheet 15 is also a sheet having a planar size for projecting from both longitudinal sides and both widthwise sides of the absorbent body 11, and similarly to the top sheet 13, because the longitudinal end portions constitute the end portion 10eLa and 10eLb of the absorbent main body 10, they have the above-mentioned approximately V-shaped tapered shape of becoming smaller in the width direction toward the front side or the back side in the longitudinal direction. The top sheet 13 and the back sheet 15 are overlaid on each other in the thickness direction so that the substantially V-like shapes at both end portions of the top sheet 13 and the back sheet 15 are substantially aligned with each other, and thus the absorbent body 11 is held between the sheets 13 and 15.

Note that as shown in FIG. 4, the back sheet 15 is formed by overlaying and joining in the thickness direction a liquid-impermeable leak-proof sheet 15f and an exterior sheet 15n that is bonded to the non-skin side of the leak-proof sheet 15f; the leak-proof sheet 15f is made of a polyethylene (PE) film, a polypropylene (PP) film, or the like, and the exterior sheet 15n is made of nonwoven fabric. However, the back sheet 15 is not limited to the above-described configuration, as long as it has a certain level of leakage resistance. Also, in this example, the planar size of the leak-proof sheet 15f is smaller than the planar size of the exterior sheet 15n, and thus the leak-proof sheet 15f fits within the outer peripheral edge of the exterior sheet 15n in the longitudinal direction and the width direction. But there is no limitation to this, and these sheets may be the same size as each other.

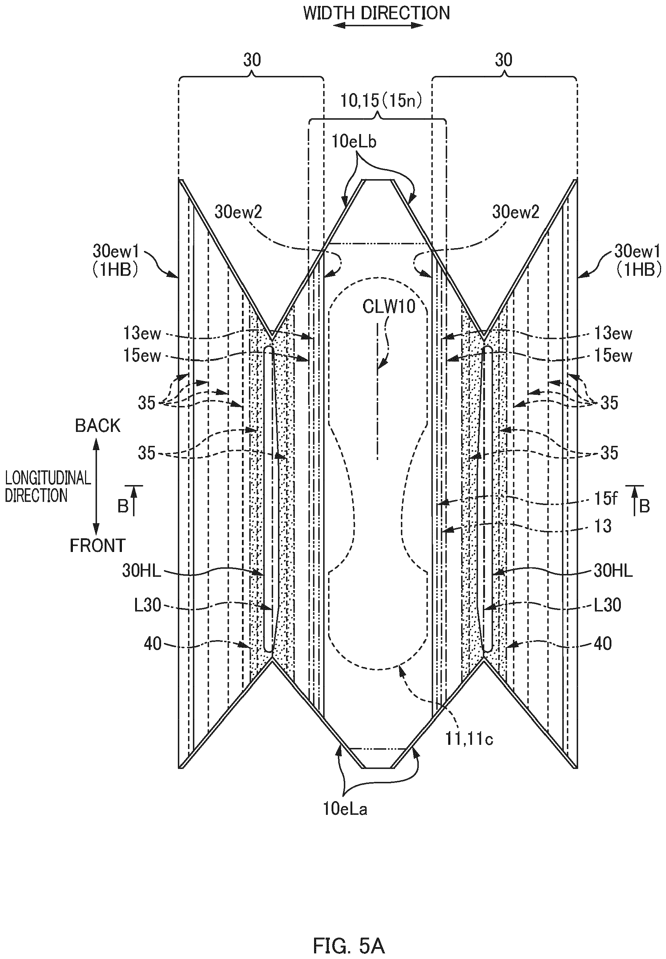

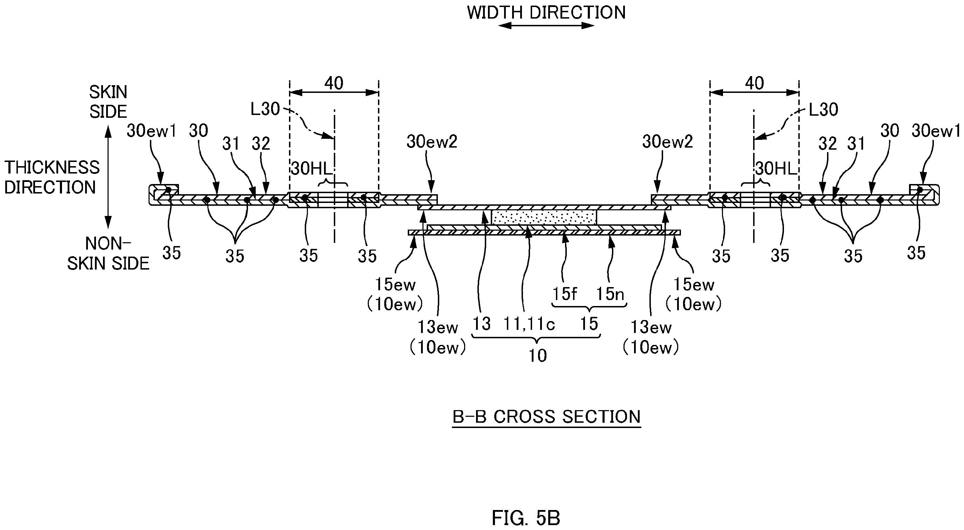

For convenience in the description, FIG. 5A is a schematic plan view of the diaper 1 in the flat extended state in FIG. 2, whose portions have been peeled and unfolded such that the skin-side surface can be seen. In other words, FIG. 5A is a schematic plan view of the diaper 1 in an unfolded state in which the attachment of the longitudinal end portions 10eLa and 10eLb of the absorbent main body 10 and the composite members 30 is broken, and the composite members 30 are opened outward in the width direction. Also, in FIG. 5A, the skin side of the absorbent main body 10 and the skin side of the composite member 30 are visible. FIG. 5B is a cross-sectional view taken along a line B-B in FIG. 5A. Note that FIGS. 5A and 5B are views in which the diaper 1 is stretched in the longitudinal direction and the width direction until the gathers are eliminated, and this also applies to FIGS. 2 to 4 described above.

As shown in FIGS. 5A and 5B, the pair of composite members 30 are sheet-like members that are elongated in the longitudinal direction, and in this example, are formed by stacking two nonwoven fabric sheets 31 and 32 in the thickness direction. Also, as described above, the pair of composite members 30 are formed by performing folding inward in the width direction from the states shown in FIGS. 5A and 5B to the states shown in FIGS. 2 and 4 at the folding lines L30 set extending along the longitudinal direction at predetermined positions in the width direction. Therefore as shown in FIGS. 2 and 4, the one composite member 30 is overlaid on the left side portion of the skin-side surface of the absorbent main body 10, and the other composite member 30 is overlaid on the right side portion of the skin-side surface. The pair of composite members 30 are in a mirror image relationship with respect to the center position CLW10 in the width direction of the absorbent main body 10. Also, in this folded state, the two widthwise end portions 30ew1 and 30ew2 of the composite member 30 are located inside in the width direction with respect to the folding line L30. Also, as shown in FIG. 4, out of these two end portions 30ew1 and 30ew2, the end portion 30ew2 closer to the absorbent main body 10 in the thickness direction is located farther outside in the width direction than the far end portion 30ew1. The closer end portion 30ew2 is joined over substantially the entire length in the longitudinal direction and using a hot-melt adhesive etc. to the widthwise end portion 13ew of the top sheet 13, which forms the skin-side surface of the absorbent main body 10. Note that in FIG. 4, it is shown that the composite member 30 is joined only to the end portion 13ew of the top sheet 13, but the present invention is not limited to this. In other words, as shown in FIG. 4, if the end portion 15ew of the back sheet 15 protrudes outward in the width direction from the end portion 13ew of the top sheet 13, the end portion 15ew of the back sheet 15 may also be joined to the composite member 30.

Also, although this has also been partly described above, as shown in FIG. 2, the two longitudinal end portions 30eLa and 30eLb of the composite members 30 are overlaid on and fixed to widthwise half portions of the substantially V-shaped end portions 10eLa and 10eLb of the absorbent main body 10. Accordingly, the shapes of the end portions 30eLa and 30eLb of the composite member 30 are inclined so as to overlap the widthwise half portions of the above-mentioned substantially V-shaped end portions 10eLa and 10eLb. Therefore the longitudinal end portions 30eLa 30eLb of the composite members 30 are overlaid on, in the thickness direction, and joined, by welding or the like, to the corresponding end portions 10eLa and 10eLb of the absorbent main body 10.

Also, in this example, when the underpants-shaped state shown in FIG. 1 is achieved, the widthwise inner end portions 30ew1 of both composite members 30 and 30 of the diaper 1 in the flat extended state shown in FIG. 2 become the waist openings 1HB. Therefore, in order to give to the end portions 30ew1 an end closing structure for reducing skin stress of the wearer, the end portions 30ew1 are folded only one time in the width direction as shown in FIG. 4 and fixed in the folded state with use of a hot-melt adhesive or the like. However, the present invention is not limited to this, and the end portions 30ew1 may be folded two or more times, or the end portions 30ew1 may not be folded.

On the other hand, as shown in FIG. 5A, the leg openings 30HL are formed so as to straddle the previously-described folding lines L30 in the width direction and pass through the composite members 30 in the thickness direction. Also, in this example, the leg openings 30HL are formed in an asymmetric shape that is not line-symmetrical with respect to the folding lines L30. Note that the asymmetrical shape is determined in consideration of the leg shape of the wearer. Therefore, in this example, the asymmetrical shape can provide a good fit to the leg of the wearer. Note that the determination of whether the shape of the leg openings 30HL is a symmetrical shape or an asymmetrical shape can be made by, for example, visually observing the shape of the leg openings 30HL in a state where the diaper 1 has been elongated in the longitudinal direction and the width direction until the gathers of the leg openings 30HL disappear in the unfolded state of FIG. 5A, or by visually observing the shape of the punching blade of a later-described die cutter device.

Also, as shown in FIGS. 5A and 5B, for the purpose of giving stretchability to the composite members 30, string-like elastic members 35 such as elastic strings that extend in the longitudinal direction are provided side-by-side in the width direction between the two nonwoven fabric sheets 31 and 32 pertaining to the composite members 30. In other words, the string-like elastic members 35 are fixed, with a hot-melt adhesive or the like, to the nonwoven fabric sheets 31 and 32 in a state of being elongated to a predetermined elongation factor in the longitudinal direction, and thus the composite members 30 are given stretchability in the longitudinal direction.

Here, in this example, some elastic members 35 among the string-like elastic members 35 are arranged in a portion between the leg openings 30HL and the waist opening 1HB in the composite members 30, but at least one other elastic member 35 is arranged in a portion of the composite members 30 between the leg openings 30HL and the absorbent main body 10. Therefore, the leg openings 30HL in the composite members 30 are subjected to contractive force in the longitudinal direction at positions on both sides in the width direction, and thus the leg openings 30HL contract while forming folds that extend along approximately the total circumference thereof, thereby forming gathers over substantially the entire circumference.

Also, in this example, as shown in FIGS. 5A and 5B, auxiliary members 40 (see the dot pattern portion in FIG. 5A) are provided for the purpose of reinforcing the leg openings 30HL. Specifically, band-shaped auxiliary members 40 having an opening that has the same shape as the leg openings 30HL are inserted between the two nonwoven fabric sheets 31 and 32 pertaining to the composite members 30, thereby reinforcing the entire circumference of the peripheral edge portions of the leg openings 30HL. Also, in this example, stretchable sheets (stretchable members) having stretchability are used as the auxiliary members 40. The auxiliary members 40 are also fixed, with a hot-melt adhesive or the like, to the nonwoven fabric sheets 31 and 32 in a state of being elongated in the longitudinal direction. Therefore, the auxiliary members 40 can also effectively contribute to the formation of gathers at the leg openings 30HL. Note that, as an example of such a stretchable sheet, it is possible to use a nonwoven fabric sheet which exhibits stretchability as follow: preparing a nonwoven fabric sheet that has substantially elastic elastomer fibers (a polyurethane elastomer etc.) and substantially non-elastic thermoplastic resin fibers (a polyolefin resin etc.), the nonwoven fabric sheet undergoes stretch processing such as gear stretching. This is used in this example, but the present invention is not limited to this. Furthermore, the auxiliary members 40 do not need to have stretchability as described above.

### Method for manufacturing diaper 1

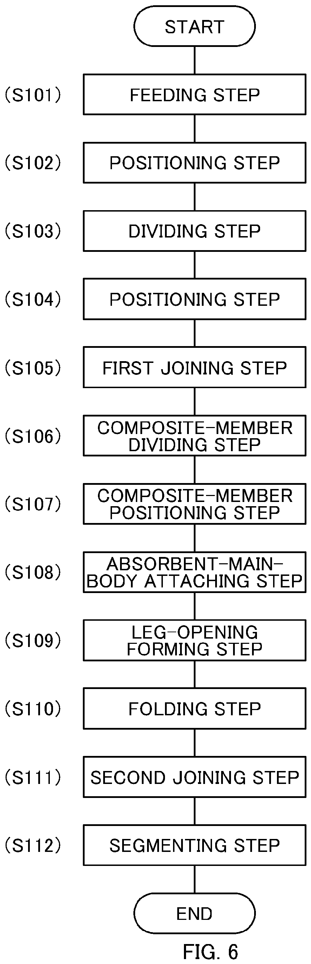

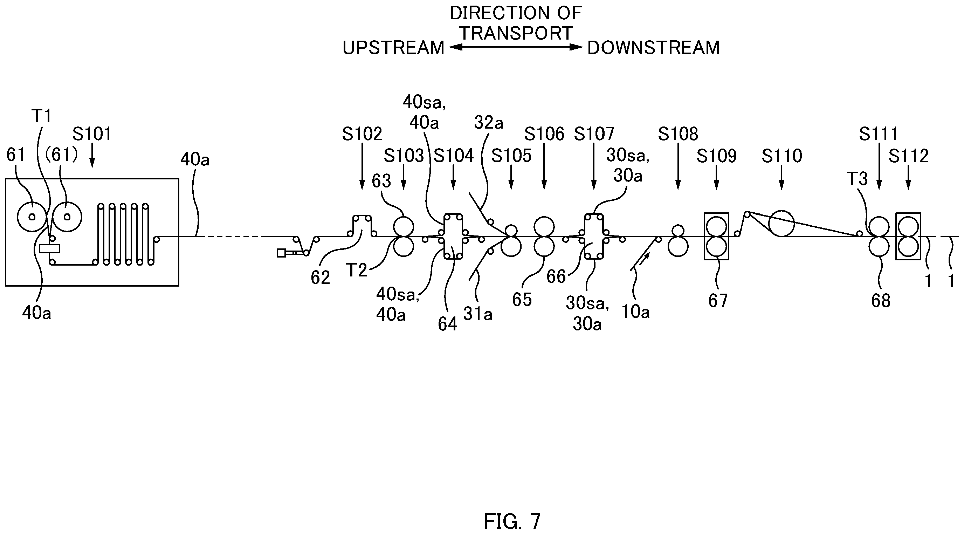

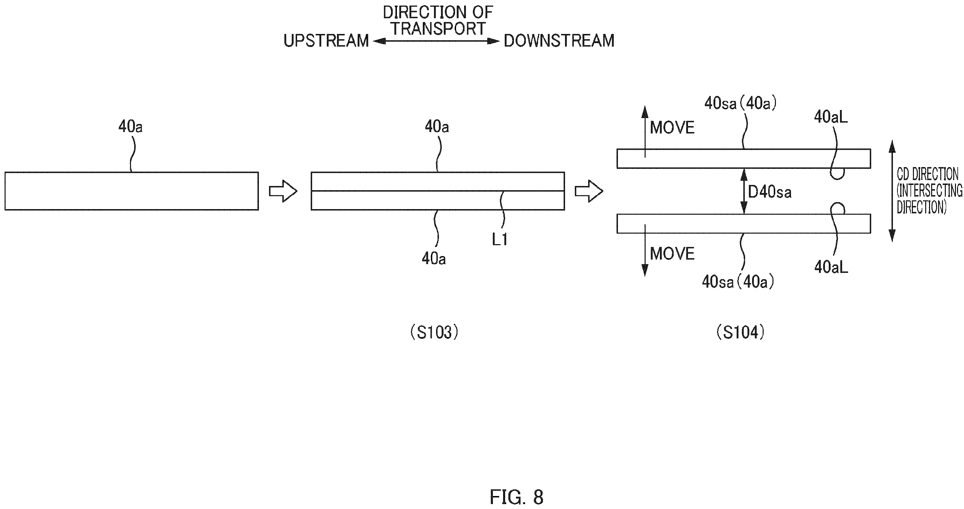

Next, a method for manufacturing the diaper 1 will be described. FIG. 6 is a flowchart of steps performed when manufacturing the diaper 1 according to the first embodiment. FIG. 7 is a schematic view showing a manufacturing line according to the first embodiment. FIGS. 8 to 12 are illustrative diagrams of the method for manufacturing the diaper 1 according to the first embodiment. Note that regarding FIGS. 6 to 12, in order to describe the present invention in an easy-to-understand manner, it is assumed that the absorbent main body 10 is supplied in a completed form, and a description will not be given for the method for manufacturing the absorbent main body 10 itself.

The diaper 1 of the present embodiment is continuously manufactured by carrying out steps S101 to S112 shown in FIG. 6 on the manufacturing line. Also, the diaper 1 is manufactured by a so-called "longitudinal flow method" on the manufacturing line. In the longitudinal flow method, while the intermediate product of the diaper 1 is transported with the longitudinal direction of the absorbent main body 10 serving as the direction of transport, various processes and the joining of other components are sequentially performed to obtain the diaper 1 in the final previously-described flat extended state. Note that in FIGS. 8 to 12, due to space limitations, a single intermediate product of a single diaper is shown, but the actual intermediate product is a continuous body or a continuous sheet that is continuous in the direction of transport.

Also, the intermediate product is transported by using a known transporting device (see FIG. 7) such as a belt conveyor (e.g., a suction belt conveyor) or conveying rollers. In the following, unless otherwise specified, it is assumed that these transporting devices are appropriately used. Also, as described above, since the direction of transport extends along the longitudinal direction of the absorbent main body 10, the direction of transport is orthogonal to the thickness direction and the width direction of the absorbent main body 10. Here, in this manufacturing line, the direction corresponding to the above-mentioned width direction is also referred to as the "CD direction". Also, the direction that intersects with the direction of transport is also referred to as the intersecting direction. The intersecting direction corresponds to the width direction described above and the CD direction. Hereinafter, the steps will be described.

### S101

First, as shown in FIG. 7, a feeding step is performed in which an auxiliary-member continuous body 40a for forming the auxiliary member 40 is unwound from a roll 61 in which the auxiliary-member continuous body 40a is wound (S101). The unwound auxiliary-member continuous body 40a is conveyed in the direction of transport.

### S102

Next, the auxiliary-member continuous body 40a is positioned at a predetermined position via a guide device 62. Specifically, a positioning step is performed in which the auxiliary-member continuous body 40a is positioned such that the central position thereof in the intersecting direction is at a predetermined position in the intersecting direction (S102). As one example, in the positioning step (S102), sensors (not shown) on both sides of the guide device 62 in the intersecting direction (width direction) detect the web (auxiliary-member continuous body 40a), and web centering is performed. As a result, in the subsequent dividing step of dividing the auxiliary-member continuous body 40a (later-described S103), the auxiliary-member continuous body 40a can be easily divided into two at the central position in the intersecting direction (width direction).

### S103

Next, as shown in FIGS. 7 and 8, a dividing step (S103) is performed in which the auxiliary-member continuous body 40a is divided at a predetermined position in the intersecting direction (CD direction). In this step, as shown in the center view of FIG. 8, first, the auxiliary-member continuous body 40a in the shown state passes through a slitter device 63 arranged at a predetermined position in the direction of transport. As it passes through the slitter device 63, the auxiliary-member continuous body 40a is divided along a dividing line L1 that is continuous in the direction of transport. The dividing line L1 is a line that passes through the central position in the CD direction, that is to say, the auxiliary-member continuous body 40a is divided into two at the central position in the CD direction. Therefore as shown in the right view of FIG. 8, a pair of narrow auxiliary-member continuous bodies 40sa, which are half the size in the CD direction, are obtained. Note that a known device having a rotary blade can be given as an example of the slitter device, but there is no limitation whatsoever to this.

### S104

Next, as shown in FIG. 7 and the right view of FIG. 8, a positioning step of positioning the divided auxiliary-member continuous bodies 40sa is performed (S104). In this step, the divided pair of narrow auxiliary-member continuous bodies 40sa pass through a spreading device 64 arranged downstream of the slitter device 63 in the direction of transport. As they pass through the spreading device 64, the narrow continuous bodies 40sa are moved outward in the CD direction, and as a result, as shown in the right view of FIG. 8, a gap D40sa is formed between the continuous bodies 40sa. Note that this is preliminary preparation for the joining of the composite members to the continuous bodies 30a, which is performed later.

Also, in this positioning step (S104), the divided auxiliary-member continuous bodies 40a are positioned based on divided ends 40aL of the auxiliary-member continuous bodies 40a in the intersecting direction when divided at the dividing line L1 in the immediately preceding dividing step (S103). In the auxiliary-member continuous body 40a, the portions that have been cut at the dividing line L1 have little variation in the width direction, and performing transporting based on the ends 40aL facilitates arrangement at the proper position in the subsequent first joining step (S105). Also, a known device having rollers tilted outward with respect to the direction of transport at positions on the two sides in the CD direction can be given as an example of the spreading device, but there is no limitation whatsoever to this.

### S105

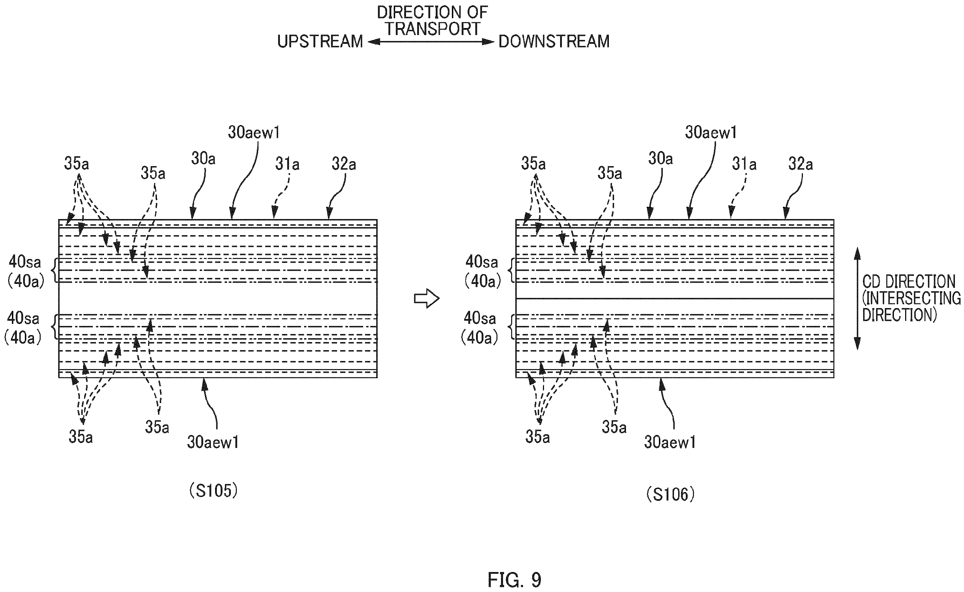

Next, as shown in FIG. 7 and the left view of FIG. 9, a first joining step (S105) is performed in which a composite-member continuous body 30a for forming the composite member 30 is joined to the auxiliary-member continuous bodies 40sa. First, as shown in FIGS. 7 and 9, the above-described two layered nonwoven fabric sheets 31 and 32 for forming the composite-member continuous body 30a are transported from the upstream step, in the form of continuous sheets 31a and 32a that are continuous in the direction of transport. Note that the "composite-member continuous body 30a" in the steps of the present embodiment is a composite-member continuous body 30a for forming the composite members 30, and includes both an aspect corresponding to the continuous body 30a in an intermediate stage up to when the waist opening 1HB and the leg openings 30HL are formed, and an aspect in which substantially a plurality of composite members 30 are continuous in the longitudinal direction.

In the first joining step (S105), as shown in the left view of FIG. 9, the divided auxiliary-member continuous bodies 40sa are stretched in the direction of transport and inserted between the two-layered nonwoven fabric continuous sheets 31a and 32a for forming the composite-member continuous body 30a, and the auxiliary-member continuous bodies 40sa and the composite-member continuous bodies 30a are joined using a hot-melt adhesive or the like.

Also, at this point, continuous bodies 35a of string-like elastic members 35 that are continuous in the direction of transport are elongated in the direction of transport and inserted between the two layered nonwoven fabric continuous sheets 31a and 32a that are for forming the composite-member continuous body 30a, and are fixed to the composite-member continuous body 30a using a hot-melt adhesive or the like.

Also, at this point, the CD-direction end portions 30aew1 of the composite-member continuous body 30a are slightly folded inward in the CD direction and fixed in the folded state using a hot-melt adhesive or the like.

### S106

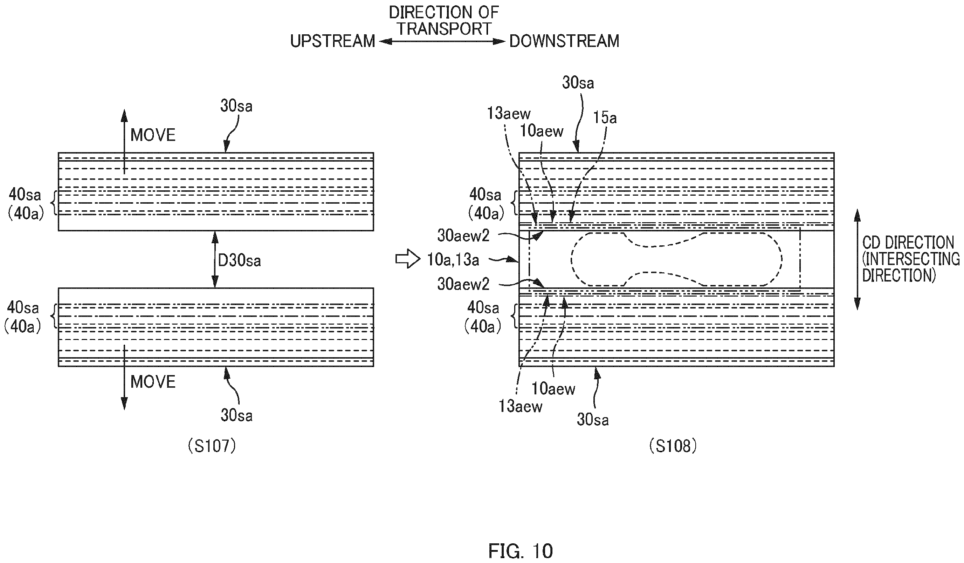

Next, as shown in FIG. 7 and the right view of FIG. 9, a dividing step (S106) is performed in which the composite-member continuous body 30a is divided at the central position in the intersecting direction (CD direction). In this step, as shown in the right view of FIG. 9, the composite-member continuous body 30a in the shown state passes through the slitter device 65 (FIG. 7) arranged at a predetermined position in the direction of transport. As it passes through the slitter device 65, the composite-member continuous body 30a is divided into two portions at the central position in the CD direction, and thus a pair of narrow composite-member continuous bodies 30sa having half the dimension in the CD direction are obtained as shown in the left view of FIG. 10.

### S107

Next, as shown in the left view of FIG. 10, a positioning step of positioning the composite-member continuous bodies 30sa is performed (S107). In this step, the pair of narrow composite-member continuous bodies 30sa pass through the spreading device 66 (FIG. 7) arranged downstream of the slitter device 65 in the direction of transport. As they pass through, the narrow composite-member continuous bodies 30sa are moved outward in the CD direction by the spreading device. As a result, as shown in the left view of FIG. 10, a gap D30sa that is slightly smaller than the CD-direction dimension of the later-described absorbent-main-body continuous body 10a is formed between the composite-member continuous bodies 30sa. Note that this is preliminary preparation for the joining of the composite-member continuous bodies 30sa to the CD-direction end portions 10aew of the absorbent-main-body continuous body 10a at the time of merging with the continuous body 10a.

### S108

Next, as shown in FIG. 7 and the right view of FIG. 10, an absorbent-main-body attaching step is performed in which the absorbent-main-body continuous body 10a for forming the absorbent main body is attached to the composite-member continuous bodies 30sa (S108). Specifically, as shown in FIG. 7, the absorbent-main-body continuous body 10a joins the transporting route of the pair of composite-member continuous bodies 30sa at a predetermined position in the direction of transport. At the time of this merging, end portions 30aew2 of the composite-member continuous bodies 30sa inside in the CD direction are joined to, using a hot-melt adhesive of the like, and integrated with CD-direction end portions 13aew of a top sheet continuous sheet 13a, the top sheet continuous sheet 13a being a sheet that forms the skin-side surface of the absorbent-main-body continuous body 10a.

### S109

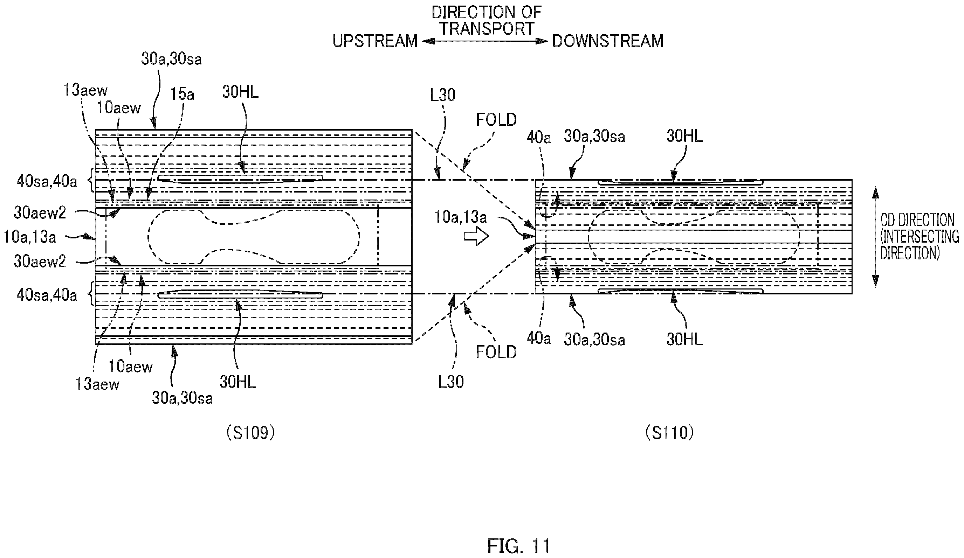

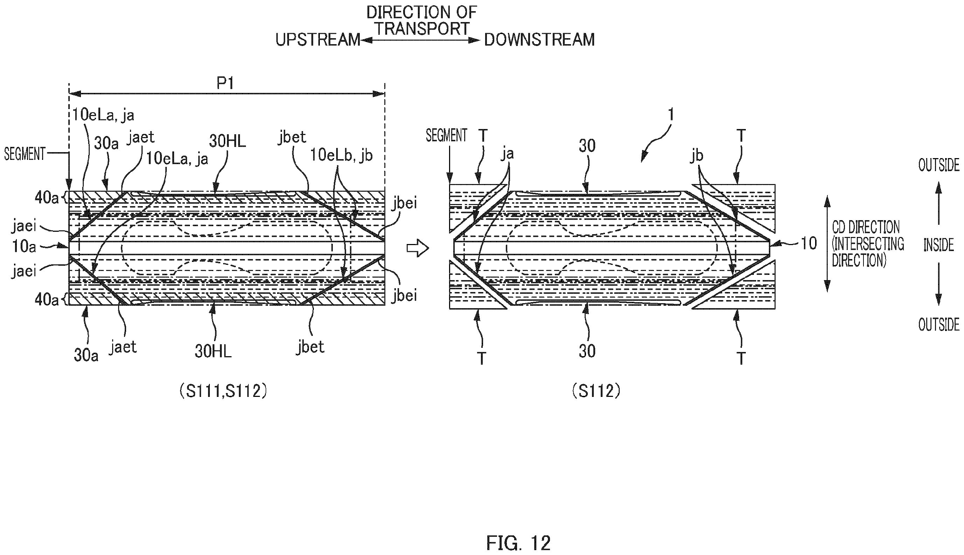

Next, as shown in the left view of FIG. 11, a leg-opening forming step is performed in which the leg openings 30HL and 30HL are each formed by performing cutting in a curved shape the auxiliary-member continuous body 40a and the composite-member continuous body 30a, which have been joined in the above-described first joining step (S109), so as to penetrate through these continuous bodies 40a and 30a. In this step, as shown in the left view of FIG. 11, a die cutter device 67 or the like is applied to the composite-member continuous body 30sa which has undergone the immediately previous absorbent-main-body attaching step (S108), and as a result the leg openings 30HL and 30HL are each formed in the composite-member continuous body 30sa at the product pitch P1 (see FIG. 12) in the direction of transport. At this point, the composite-member continuous body 30sa and 30sa become a pair of composite-member continuous bodies 30a and 30a in each of which a plurality of composite members 30 are continuous in the longitudinal direction.

Note that here, as shown in FIG. 11, at the time of forming each leg opening 30HL, the above-described folding line L30 of the composite member 30 has also been set in the composite-member continuous body 30sa (30a), and as a result, the die cutter device 67 forms the leg opening 30HL so as to straddle the folding line L30 in the CD direction (intersecting direction). Also, the time at which the leg opening 30HL is formed is a time before the composite-member continuous body 30sa (30a) is folded back at the folding line L30. Therefore, the asymmetrical leg opening 30HL can be formed in the composite-member continuous body 30sa (30a) without any problem, thereby increasing the degree of freedom in selecting the shape of each of the leg openings 30HL.

Also, at the time of forming the leg openings 30HL in the composite-member continuous bodies 30a, the auxiliary members 40 have already been provided, in the form of the continuous bodies 40a, in the composite-member continuous bodies 30sa. For this reason, the leg openings 30HL are formed so as to penetrate not only the composite-member continuous bodies 30sa but also the auxiliary member continuous sheets 40a.

Also, a known device having a punching blade type of cutter roller and an anvil roller can be given as an example of the die cutter device 67, but the present invention is not limited to this.

### S110

Next, as shown in FIG. 7 and the right view of FIG. 11, a folding step is performed in which the pair of composite-member continuous bodies 30a and 30a are folded inward in the CD direction (intersecting direction) at the folding lines L30 and L30 set in the continuous bodies 30a and 30a (S110). Accordingly, the composite-member continuous bodies 30a and 30a respectively located on one side and the other side in the CD direction are respectively overlaid on the CD-direction one-side portion and the CD-direction other-side portion, of the skin-side surface of the absorbent-main-body continuous body 10a. Also, in this folding step (S110), the auxiliary-member continuous bodies 40a and 40a are folded together with the composite-member continuous bodies 30a and 30a in the intersecting direction (CD direction).

This folding process is performed by a known folding device. Specifically, when the pair of composite-member continuous bodies 30a that has been joined to the absorbent-main-body continuous body 10a pass through the positions of the folding plate members of the folding device arranged at a predetermined position in the direction of transport, the folding plate members come into contact at or near the folding lines L30 of the pair of composite-member continuous bodies 30a or a portion in the vicinity thereof, and thus the pair of composite-member continuous bodies 30a are folded at the folding lines L30.

### S111

Next, as shown in the left view of FIG. 12, a second joining step is performed in which the auxiliary-member continuous bodies 40a and the composite-member continuous bodies 30a, which were joined in the first joining step (S105) described above, are joined to the absorbent-main-body continuous body 10a to form joining portions ja and jb that intersect the direction of transport (S111). In this step, the pair of composite-member continuous bodies 30a are fixed in the state of having been folded in the folding step (S110) described above.

As shown in the left view of FIG. 12, such fixing is performed by joining the absorbent-main-body continuous body 10a and the pair of composite-member continuous bodies 30a and 30a at positions that correspond to the front end portion 10eLa and the back end portion 10eLb of the absorbent main body 10. This joining processing can be performed by a known heat sealing device 68 that has an embossing roller and an anvil roller. Specifically, when the absorbent-main-body continuous body 10a, on which the pair of composite-member continuous bodies 30a and 30a are overlaid, passes through the position of the heat sealing device 68 in the direction of transport, with use of protrusions on the outer peripheral surface of the embossing roller and the outer peripheral surface of the anvil roller, the absorbent-main-body continuous body 10a and the pair of composite-member continuous bodies 30a are crimped together in the thickness direction and joined by welding or the like at positions corresponding to the stomach-side end portion 10eLa and the backside end portion 10eLb of the absorbent main body 10. Substantially V-shaped joining portions ja and jb are formed, as marks formed by the joining, at positions corresponding to the front end portion 10eLa and the back end portion 10eLb of the absorbent main body 10. In other words, through this step, in the absorbent-main-body continuous body 10a, a pair of joining portions ja (corresponding to a pair of first joining portions) are formed on the upstream side (corresponding to the front side) in the direction of transport, and a pair of joining portions jb (corresponding to a pair of second joining portions) are formed on the downstream side (corresponding to the back side).

Here, in the case where the joining portions ja and jb are formed by welding the materials, if the weld portions where the materials are welded are formed continuously within the range of the joining portions ja and jb or without gaps in the entire region, there is a risk that the joining portions ja and jb become hard, and there is a risk that wrinkles form in the absorbent-main-body continuous body 10a. In that respect, in the present embodiment, a plurality of weld portions (not shown) for welding materials are intermittently provided. Also, as shown in the left view of FIG. 12, the joining portions ja (first joining portions) are provided so as to be inclined toward the center from outer sides in the intersecting direction, and the joining portions jb (second joining portions) are provided so as to be inclined outward in the intersecting direction from the center. In the case where a plurality of weld portions are provided intermittently, if the weld portions are formed in only one direction, it causes a risk that wrinkles easily form in the absorbent-article continuous body 10a due to a continuous force that acts in the one direction. In the present embodiment, a force acts inward in the intersecting direction (width direction) in the joining portions ja (first joining portions), and a force acts outward in the intersecting direction in the joining portions jb (second joining portions). This makes it possible to apply force alternatingly to the absorbent-article continuous body 10a, making wrinkles less likely to form.

### S112

Finally, as shown in the left and right views of FIG. 12, a segmenting step (S112) is performed in which while the absorbent-main-body continuous body 10a, to which the pair of composite-member continuous bodies 30a have been joined by the substantially V-shaped joining portions ja and jb described above, is transported in the direction of transport, the absorbent-main-body continuous body 10a is segmented at the product pitch P1 of the diaper 1, and substantially triangular ends T are cut away at positions near the substantially V-shaped joining portions ja and jb. As a result, the diaper 1 in the flat extended state shown in FIG. 2 is obtained. Note that the segmenting/cutting processing can be performed by a known cutter device that has a cutter roll and an anvil roll, but the present invention is not limited to this.

In the above-described steps S101 to S112 of the present embodiment, first one auxiliary-member continuous body 40a is fed, divided, and then joined to the composite-member continuous bodies 30a. Conventionally, when an auxiliary member made of a continuous web sheet of a stretchable member or a reinforcing member is arranged on a pair of members (a pair of composite members 30) that have leg and waist openings, there are cases where two auxiliary-member continuous bodies for forming the auxiliary members are prepared and respectively arranged on the pair of composite members. However, in the case where the auxiliary member is a stretchable member or the like, the auxiliary member wound around the roll is often not stable due to slight variation in its width or due to deformation or misalignment of the widthwise end portions of the auxiliary member. Accordingly, in the present embodiment, such variation can be reduced by dividing the one auxiliary-member continuous body 40a into two at the central position in the CD direction (width direction). Note that the dividing position is not limited to being the central position in the CD direction, and dividing may be performed at a predetermined position in the CD direction.

Accordingly, the arrangement position of the auxiliary-member continuous body 40a with respect to the range of the formed joining portions ja and jb is stable in the second joining step (Sill) in which the auxiliary-member continuous body 40a is finally joined together with the composite-member continuous bodies 30a and the absorbent-main-body continuous body 10a. Specifically, at the joining positions (positions of the weld portions) in the joining portions ja and jb, the number of the overlaid materials is stable and does not vary. As a result, the joining strength of the joining portions ja and jb can be easily stabilized. Also, as described above, in the case where two auxiliary-member continuous bodies 30a are introduced, problems such as the need for excessive equipment space and complicated control are likely to occur. But in the present embodiment, the supply of the sheet for forming the auxiliary members is performed by supplying one sheet (auxiliary-member continuous body 40a), and therefore it is possible to save equipment space and reduce the complexity of device control.

Also, in the present embodiment, the dividing step (S103) is performed between the feeding step (S101) and the first joining step (S105), but the present invention is not limited to this, and it is sufficient that the dividing step (S103) is performed between the feeding step (S101) and the second joining step (S111).

Here, in the second joining step (Sill) of the present embodiment, as shown in the left view of FIG. 12, the auxiliary-member continuous body 40a is arranged so as to overlap outer end portions jaet and jaet of the joining portions ja and outer end portions jbet and jbet of the joining portions jb, the outer end portions jaet, jaet, jbet, and jbet are located closer to outside in the intersecting direction. In the product state, the outer end portions jaet and jbet of the joining portions ja and jb are included in the leg openings 30HL (see FIG. 1) and are portions that require strength. Accordingly, the auxiliary-member continuous body 40a that has been divided in the dividing step (S103) of the present embodiment has a reduced amount of widthwise variation in the width direction (intersecting direction), and therefore even when the joining portions ja and jb are formed in the second joining step (Sill), it is possible to stably arrange the auxiliary-member continuous body 40a up to the outer end portions jaet and jbet of the joining portions ja and jb. Note that in the present embodiment, the auxiliary-member continuous body 40a is arranged at the outer end portions jaet and jbet of the joining portions ja and jb, but the present invention is not limited to this. The auxiliary-member continuous body 40a may be arranged so as to overlap at least either the intersecting-direction outer end portions jaet and jbet or intersecting-direction inner end portions jaei and jbei of the joining portions ja and jb. Since the inner end portions jaei and jbei are included in the waist opening 1HB in the product state (see FIG. 1), the inner end portions jaei and jbei are also portions that require strength. Even in the case where the auxiliary-member continuous body 40a is arranged in such portions, it can similarly be stably arranged up to the inner end portions jaei and jbei.

Also, as described above, rather than being limited to being arranged on at least either the intersecting-direction outer end portions jaet and jbet or the intersecting-direction inner end portions jaei and jbei of the joining portions ja and jb, in the second joining step (Sill), the auxiliary-member continuous body 40a may be arranged on both the outer end portions jaet and jbet and the inner end portions jaei and jbei of the joining portions ja and jb in the intersecting direction. According to this configuration, it increases the number of constituent members that are overlaid at the two end portions (the outer end portions jaet (jbet) and the inner end portions jaei (jbei)) of the joining portions ja (jb), making it easier to more reliably form the joining portions ja (jb) at the two end portions. This therefore makes it easier to stabilize the quality of the product.

Also, in the present embodiment, the folding step (S110) is performed before the second joining step (Sill). In the folding step (S110), the auxiliary-member continuous body 40a is folded together with the composite-member continuous body 30a in the intersecting direction. Then, in the subsequent second joining step (Sill), the joining portions ja and jb are formed such that an overlapping portion (portion shown by hatching in the left view of FIG. 12), where portions of the auxiliary-member continuous body 40a are overlapped due to the folding step (S110), is arranged in the intersecting-direction outer end portions jaet and jbet of the joining portions ja and jb.

Note that the case where the auxiliary-member continuous body 40a is to be arranged on the intersecting-direction inner end portions jaei and jbei of the joining portions ja and jb is the case where in the first joining step (S105), the auxiliary-member continuous bodies 40sa and 40sa are arranged so as to overlap the CD-direction end portions 30aew1 and 30aew1 (right view of FIG. 9) of the composite-member continuous body 30a. The end portions 30aew1 are folded inward in the CD direction (intersecting direction) in the folding step (S110), and in the diaper 1 in the flat extended state of FIG. 2, these end portions are located in the center in the width direction and form the waist opening 1HB. Also, because the end portions 30aew1 and 30aew1 of the composite-member continuous body 30a in the CD direction are folded inward in the CD direction as described above, arranging the auxiliary-member continuous body 40a at the end portions 30aew1 forms an overlapping portion where portions of the auxiliary-member continuous body 40a are overlapped with each other. According to this configuration, finally, in the second joining step (Sill), the joining portions ja and jb are formed such that the overlapping portions are located at the intersecting-direction inner end portions jaei and jbei of the joining portions ja and jb.

Here, in the case where the variation in the width direction originally occurs in the continuous sheet that corresponds to the auxiliary-member continuous body 40a as in the conventional case, if such a continuous sheet is folded, there is a risk of the occurrence of further variation in the arrangement position of the continuous sheet. However, because the variation in the width direction is reduced in the auxiliary-member continuous body 40a that has been divided in the dividing step (S103) as in the present embodiment, even if the auxiliary-member continuous body 40a is folded and in the overlapped state when arranged on the end portions (jaet and jbet or jaei and jbei) of the joining portions ja and jb, it is possible to reduce the amount of variation in such an arrangement. In other words, the number of constituent members that are overlapped in the thickness direction in the end portions jaet, jbet, jaei, and jbei is stable, and the joining strength of the end portions jaet, jbet, jaei, and jbei is easily stabilized.

Also, in the above-described leg-opening forming step (S109), the leg openings 30HL and 30HL are each formed so as to straddle the folding line L30 in the intersecting direction, and the auxiliary-member continuous body 40a is also folded at the folding line L30. Normally, when forming the curved leg opening 30HL, it is sometimes difficult to properly arrange the auxiliary member 40 at the ends of the curved leg openings 30HL. In this respect, in the case of the present embodiment, it is possible to reduce the variation in the position of the auxiliary-member continuous body 40a arranged at the ends of the leg openings 30HL in the width direction (intersecting direction), making it easier to properly arrange the auxiliary member 40 over the entire circumference of the ends of the leg openings 30HL.

Also, the auxiliary member 40 of the present embodiment has stretchability, and it is preferable that the amount of elongation per unit length of the auxiliary-member continuous body 40a in the above-described dividing step (S103) is smaller than the amount of elongation per unit length of the auxiliary-member continuous body 40a in the above-described first joining step (S105). The amount of elongation here means the amount of elongation from the natural length of the auxiliary-member continuous body 40a. The amount of elongation per unit length is an amount obtained by the amount of elongation of the auxiliary-member continuous body 40a from the natural length being divided by the natural length. When the auxiliary-member continuous body 40a is divided in the dividing step (S103), the elongation of the auxiliary-member continuous body 40a is suppressed when performing division so as to reduce the variation in size in the width direction. On the other hand, in the first joining step (S105), the auxiliary-member continuous body 40a, which has little variation in width, is joined to the composite-member continuous body 30a in the elongated state, thus making it easier to stabilize the position of the auxiliary-member continuous body 40a in the width direction.

Also, returning to FIG. 7, in the present embodiment, letting T1 be the position where the auxiliary-member continuous body 40a is fed from the roll in the feeding step (S101), letting T2 be the position where the auxiliary-member continuous body 40a is divided in the dividing step (S103), and letting T3 be the position of joining in the second joining step (Sill), it is preferable that the transporting distance in the manufacturing process from when the auxiliary-member continuous body 40a is divided in the dividing step (S103) to when joining is performed in the second joining step (S111), that is to say the transporting distance between T2 and T3, is shorter than the transporting distance from when the auxiliary-member continuous body 40a is fed from the roll to when the dividing is performed in the dividing step (S103), that is to say the transporting distance between T1 and T2. Specifically, the auxiliary-member continuous body 40a in the wide state before being divided can be stably transported even if the auxiliary member has variation in the width. After the width of the auxiliary-member continuous body 40a (40sa) is reduced due to division, using a shorter transporting distance and minimizing the variation in width after division makes it possible to maintain stability.

### Second Embodiment

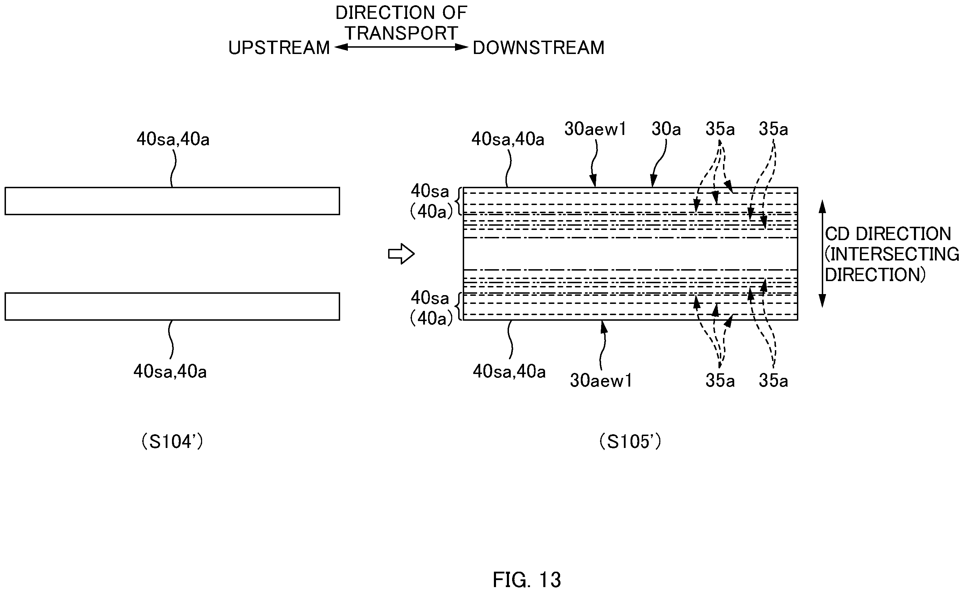

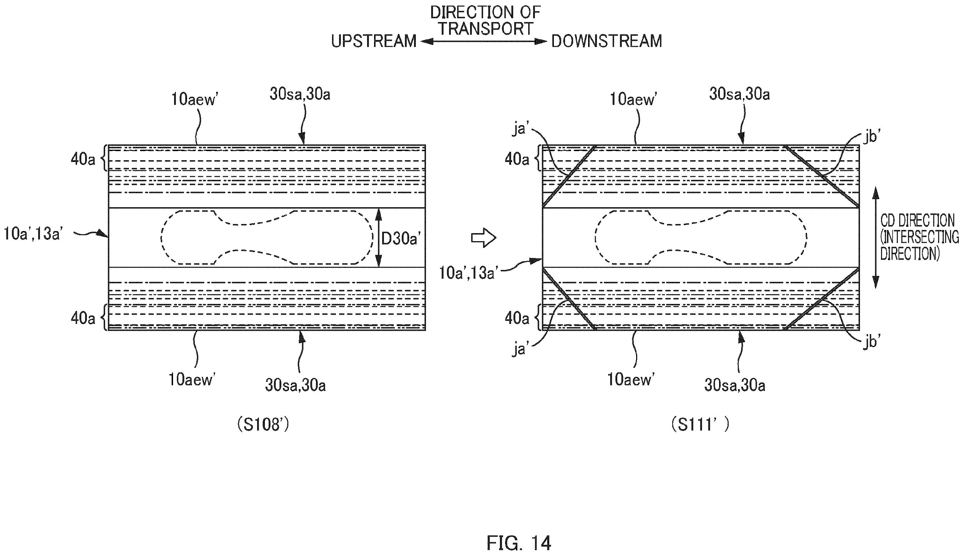

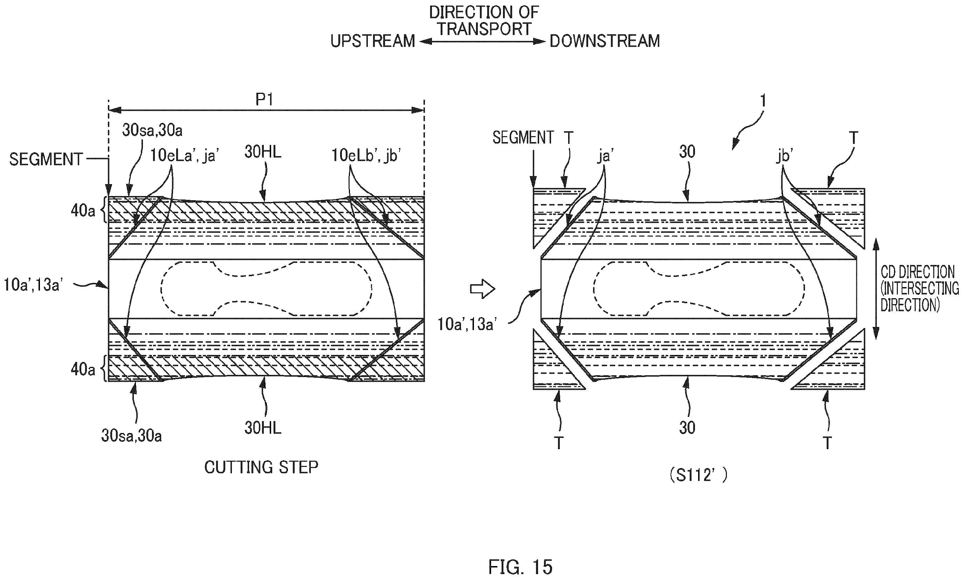

FIG. 13 is an illustrative diagram of a positioning step and a first joining step of a manufacturing method according to a second embodiment. FIG. 14 is an illustrative diagram of an absorbent-main-body attaching step and a second joining step of the second embodiment. FIG. 15 is an illustrative diagram of a cutting step and a segmenting step of the second embodiment.

The main differences from the first embodiment are that the arrangement position of the auxiliary-member continuous body 40a with respect to the composite-member continuous body 30a in the first joining step (S105) is different, the leg-opening forming step (S109) is not included, a cutting step is performed, and the folding step (S110) is not performed. Also, the leg openings 30HL of the diaper 1 manufactured by the manufacturing method of the first embodiment are constituted by a pair of composite members, but the diaper 1 manufactured by the manufacturing method of the second embodiment is a so-called three-piece type of diaper, and the leg openings 30HL are constituted by the pair of composite members 30 and the absorbent main body 10. Other than this, the configuration is almost the same as that of the first embodiment. Therefore, in the following, the differences will be mainly described, and the same reference numerals will be given to configurations that are the same as those in the first embodiment, and the description thereof will be omitted.

As shown in FIG. 13, a positioning step (S104') of positioning the divided auxiliary-member continuous bodies 40sa is performed, and then a first joining step (S105') of joining the composite-member continuous body 30a to the auxiliary-member continuous bodies 40sa is performed. In the first joining step, unlike the first embodiment, the auxiliary-member continuous bodies 40sa are arranged at positions at the two end portions of the composite-member continuous body 30a in the CD direction (intersecting direction).

After performing a dividing step (S106', not shown) for dividing the composite-member continuous body 30a at the central position in the intersecting direction (CD direction), the composite-member continuous bodies 30sa are positioned (positioning step S107, not shown), and then an absorbent-main-body attaching step (S108') is performed, as shown in the left view of FIG. 14. In the immediately previous positioning step, there is a gap D30a' (see FIG. 14) in the CD direction between the composite-member continuous bodies 30sa, and in the absorbent-main-body attaching step (S108'), and the pair of composite-member continuous bodies 30sa are arranged such that length between the outer ends thereof in the CD direction is substantially the same as the length of the absorbent-main-body continuous body 10a' in the CD direction.

Next, a second joining step (S111') is performed in which the auxiliary-member continuous bodies 40a, the composite-member continuous body 30a, and the absorbent-main-body continuous body 10a' are joined by forming joining portions ja' and jb' that intersect the direction of transport.

In the second embodiment, after the second joining step (S111'), as shown in the left view of FIG. 15, a cutting step is performed in which the auxiliary-member continuous bodies 40a and 40a are cut together with the composite-member continuous bodies 30a and 30a along cutting lines (not shown) that are respectively set in the composite-member continuous bodies 30a and 30a and extends in a curved shape along the direction of transport. In this cutting step, the absorbent-main-body continuous body 10a' is cut together with the composite-member continuous bodies 30a and 30a, thus forming the leg openings 30HL. Then, the auxiliary-member continuous body 40a is arranged at a portion that is constituted by the composite-member continuous body 30a and that is in edge portions of the curved leg opening 30HL formed by the cutting step. In other words, in a stage before the second joining step (S111'), the variation in the width direction of the auxiliary-member continuous body 40a is reduced and the auxiliary member is stably arranged. Therefore even when the composite-member continuous body 30a and the auxiliary-member continuous body 40a are cut in a curved shape to form the leg opening 30HL, the auxiliary member 40 can be appropriately arranged up to the edge portion of the leg opening 30HL.

### Third Embodiment

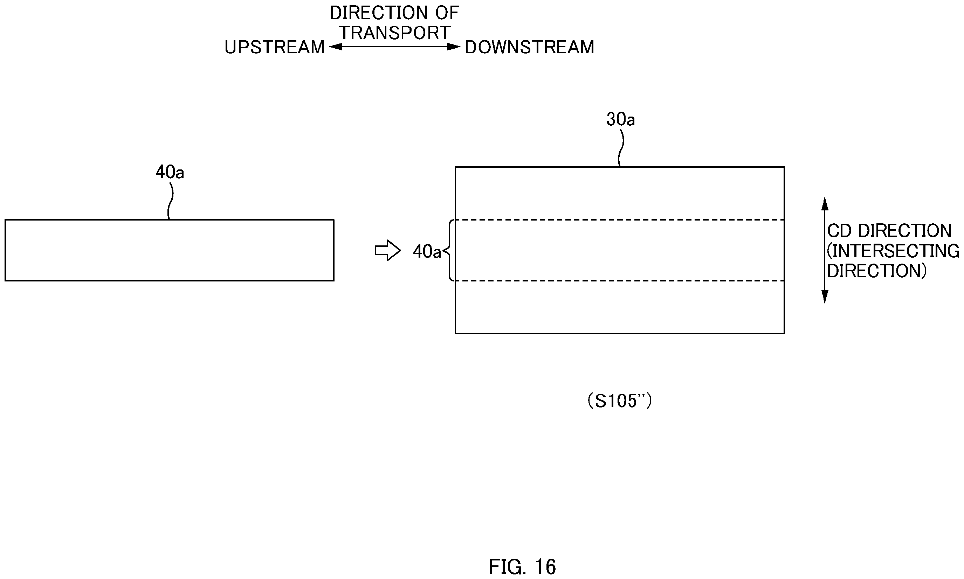

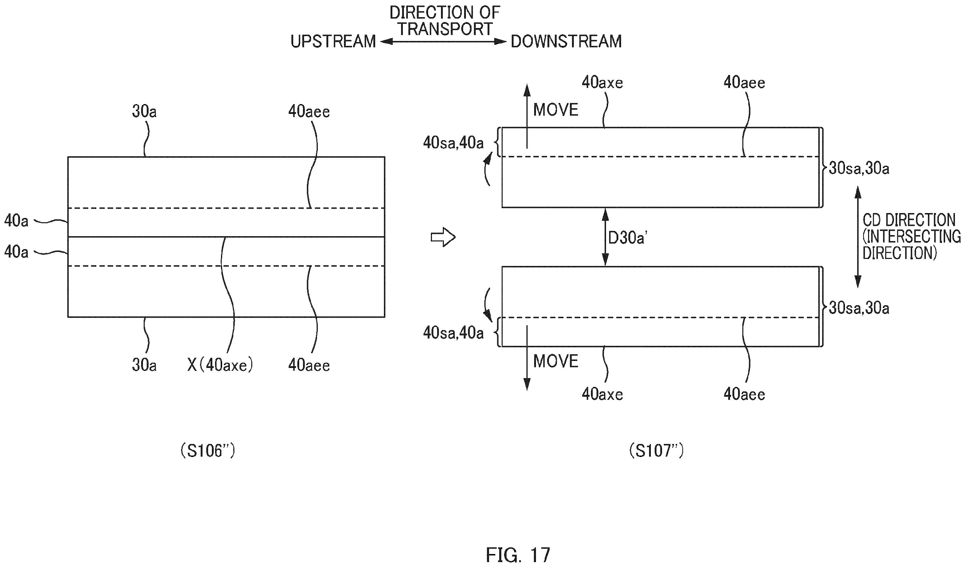

FIG. 16 is an illustrative diagram of a first joining step of a manufacturing method according to a third embodiment. FIG. 17 is an illustrative diagram of a composite-member dividing step and a composite-member positioning step of the manufacturing method of the third embodiment. Note that in FIGS. 16 and 17, for convenience in the description, the plurality of string-like elastic members 35 shown in FIGS. 9 and 10 and the like are omitted.

A main difference from the first and second embodiments is that the dividing step (S103) for dividing the auxiliary-member continuous body 40a is performed after the first joining step (S105). Also, in the third embodiment, the steps performed after the composite-member positioning step (S107') are similar to those in the second embodiment. Accordingly, in the following, this difference will be mainly described, and the same reference numerals will be given to configurations that are the same as those of the first embodiment (and the second embodiment), and the description thereof will be omitted.

As shown in FIG. 16, the auxiliary-member continuous body 40a that is fed from the roll and transported in the direction of transport is first joined to the composite-member continuous body 30a (S105", corresponding to the first joining step). In this step, as shown in the right view of FIG. 16, the auxiliary-member continuous body 40a is arranged at the central portion of the composite-member continuous body 30a in the CD direction.

Next, as shown in the left view of FIG. 17, a composite-member dividing step (S106") of dividing the composite-member continuous body 30a at the central position in the CD direction is performed. In this composite-member dividing step, the auxiliary-member continuous body 40a is divided together with the composite-member continuous body 30a along a dividing line X that is continuous in the direction of transport. Specifically, in the third embodiment, the dividing step (S103) of the first and second embodiments is included in the composite-member dividing step (S106").

In this way, the dividing step (S103) for dividing the auxiliary-member continuous body 40a is performed after the first joining step (S105"), that is to say, the auxiliary-member continuous body 40a and the composite-member continuous body 30a are first joined, and then the auxiliary-member continuous body 40a is divided. This makes it possible to arrange the auxiliary member 40 more appropriately with respect to the composite member 30.

Next, as shown in the right view of FIG. 17, a positioning step of positioning the composite-member continuous bodies 30sa and 30sa is performed (S107"). In the positioning step (S107") of the third embodiment, the left and right sides of the pair of composite-member continuous bodies 30sa and 30sa, which have been divided in the composite-member dividing step (S106"), are switched by being flipped over, for example. Specifically, in the composite-member dividing step (S106"), letting ends 40aex of the auxiliary-member continuous bodies 40a that have been divided at the dividing line X be the ends that have been divided in the intersecting direction (CD direction), and letting ends 40aee be the ends on the sides that have not been divided, the ends 40axe are located at the center in the CD direction in the composite-member dividing step (S106"). Then, in the positioning step (S107"), the ends 40aex and 40aex are arranged so as to be on opposite sides in the CD direction. According to this configuration, in the downstream cutting step that is similar to that in the second embodiment, cutting of the two sides in the CD direction are made along cutting lines that extend in a curved shape in the direction of transport, forming the leg openings 30HL. Thus the end portions 40aex and 40aex are arranged at the ends of the leg openings 30HL and 30HL. As a result, the auxiliary members 40 can be arranged very nearly up to the ends of the leg openings 30HL constituted by the composite members 30.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof.

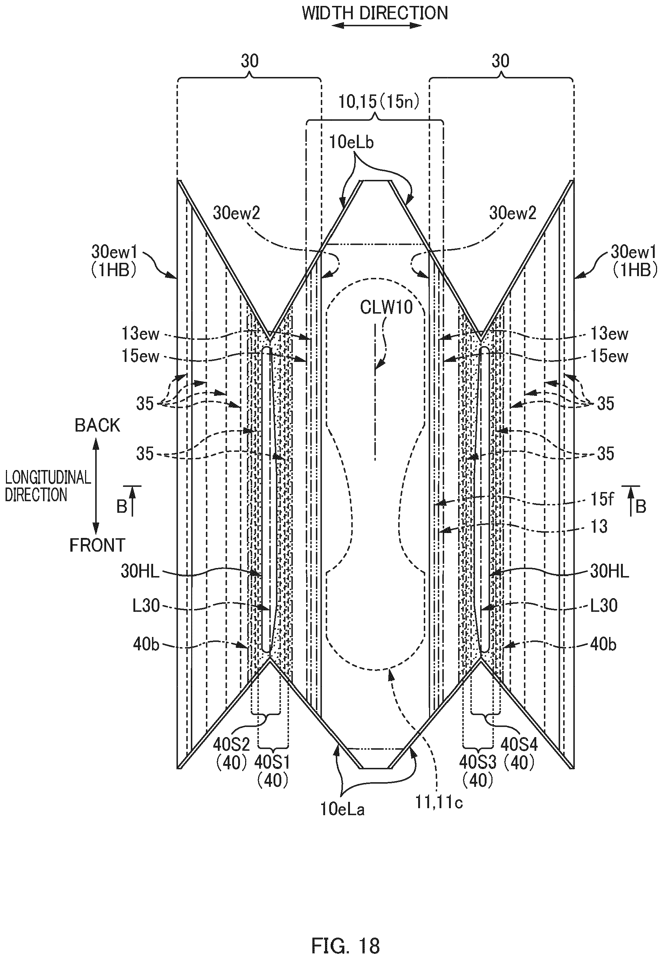

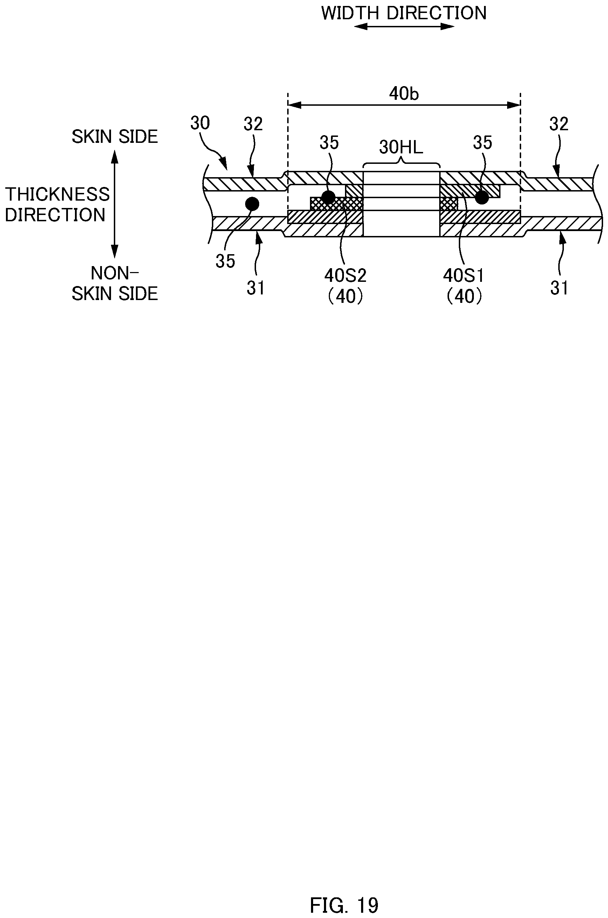

FIG. 18 is a schematic plan view of the diaper 1 in the flat extended state in a variation, whose portions have been peeled and unfolded such that the skin-side surface can be seen. FIG. 19 is a diagram showing a portion of a cross-sectional view taken along a line B-B in FIG. 18.

In the first embodiment, the auxiliary-member continuous body 40a is divided into two portions in the dividing step (S103), but in this variation, in the dividing step (S103), the auxiliary-member continuous body 40a is divided into at least four portions at a plurality of predetermined positions in the intersecting direction. Also, in the first joining step (S105), the auxiliary-member continuous bodies 40a are arranged such that, in each of two pairs of the four auxiliary-member continuous bodies 40S1, 40S2, 40S3, and 40S4, the two auxiliary-member continuous bodies have an overlapping portion before being joined to the composite-member continuous bodies 30a.

As shown in FIG. 18, in this variation, the auxiliary-member continuous bodies 40S1 and 40S2 are arranged at positions shifted to the left and right in the width direction, and the auxiliary-member continuous bodies 40S1 and 40S2 are arranged so as to have a portion where they overlap. Also, the auxiliary-member continuous bodies 40S3 and 40S4 are similarly arranged at positions shifted to the left and right in the width direction, and the auxiliary-member continuous bodies 40S3 and 40S4 are arranged so as to have a portion where they overlap. Here, the auxiliary members 40 are reinforcing members that reinforce the leg openings 30HL, and as shown in FIG. 19, stretchable members 40b are further arranged on the non-skin side in the thickness direction relative to the auxiliary-member continuous bodies 40S1, 40S2, 40S3, and 40S4. As a result, it is possible to improve the fit while also reinforcing the leg openings 30HL. Also, in the case where the first joining step is performed after the auxiliary-member continuous body 40a is divided into four portions as in this variation, it is possible to optimize the arrangement of the constituent materials of the joining portions ja and jb that are formed in the final second joining step, and the strength of the joining portions ja and jb can be further stabilized. Note that although the auxiliary-member continuous body 40a is divided into four portions in this variation, the present invention is not limited to this, and the continuous body 40a may be divided into any number of portions.

### Reference Signs

1 disposable diaper (absorbent article)
1HB waist opening
10 absorbent main body, 10ew end portion,
10eLa end portion, 10eLb end portion,
10a absorbent-main-body continuous bod, 10aew end portion,
11 absorbent body, 11c absorbent core,
13 top sheet, 13ew end portion,
13a top sheet continuous sheet, 13aew end portion,
15 back sheet, 15ew end portion,
15a back sheet continuous sheet,
15f leak-proof sheet,
15n exterior sheet,
30 composite member, 30a composite-member continuous body,
30ew1 end portion, 30ew2 end portion,
30eLa end portion, 30eLb end portion,
30aew2 end portion, 30sa composite-member continuous body,
30aew1 end portion, 31 nonwoven fabric sheet,
31a nonwoven fabric continuous sheet, 32 nonwoven fabric sheet,
32a nonwoven fabric sheet continuous sheet,
30HL leg opening,
35 string-like elastic member, 35a string-like elastic member continuous body,
40 auxiliary member, 40a auxiliary-member continuous body, 40aL end,
40aee end, 40aex end,
40b elastic member, 40S1~40S4 auxiliary-member continuous body,
61 roll, 62 guide device, 63 slitter device,
64 spreading device, 65 slitter device, 66 spreading device,
67 die cutter device, 68 heat sealing device,
L30 folding line, T end,
ja joining portion (first joining portion), jb joining portion (second joining portion),
jaei inner end portion, jaet outer end portion,
jbei inner end portion, jbet outer end portion,
CLL10 substantially central position, CLL30 substantially central position,
CLW10 center position

## Claims

1. A method for manufacturing an absorbent article (1),
the absorbent article (1) including an absorbent main body (10), a pair of composite members (30), and an auxiliary member (40),
the absorbent main body (10) being for absorbing a liquid,
the pair of composite members (30) having leg openings (30HL) and a waist opening (1HB),
the auxiliary member (40) being provided on each of the composite members (30),
the method comprising:
a feeding step (S101) of feeding an auxiliary-member continuous body (40a) from a roll (61),
the auxiliary-member continuous body (40a) being for forming the auxiliary member (40),
the auxiliary-member continuous body (40a) being wound around the roll (61);
a first joining step (S105) of joining a composite-member continuous body (30a) and the auxiliary-member continuous body (40a) that has been fed from the roll,
the composite-member continuous body (30a) being for forming the composite member (30);
a second joining step (Sill) of joining an absorbent-main-body continuous body (10a) to the auxiliary-member continuous body (40a) and the composite-member continuous body (30a) that have been joined in the first joining step (S105),
the second joining step (Sill) being performed by forming a joining portion (ja, jb),
the joining portion (ja, jb) intersecting a direction of transport,
the absorbent-main-body continuous body (10a) being for forming the absorbent main body (10); and
a dividing step (S103) of dividing the auxiliary-member continuous body (40a) at a predetermined position in an intersecting direction,
the dividing step (S103) being performed between the feeding step (S101) and the second joining step (Sill),
the intersecting direction being a direction that intersects the direction of transport.

2. A method for manufacturing an absorbent article (1) according to claim 1, wherein
the auxiliary member (40) is a stretchable member.

3. A method for manufacturing an absorbent article (1) according to claim 1 or 2, wherein
in the second joining step (Sill),
the auxiliary-member continuous body (40a) is arranged so as to overlap at least one of an intersecting-direction outer end portion (jaet, jbet) and an intersecting-direction inner end portion (jaei, jbei) of the joining portion (ja, jb) that is to be formed,
the intersecting-direction outer end portion (jaet, jbet) being an end portion located closer to outside in the intersecting direction,
the intersecting-direction inner end portion (jaei, jbei) being an end portion located closer to inside in the intersecting direction.

4. A method for manufacturing an absorbent article (1) according to claim 3, wherein
the method further comprises a folding step (S110) of folding the composite-member continuous body (30a) in the intersecting direction at a folding line (L30),
the folding step (S110) being performed between the first joining step (S105) and the second joining step (Sill),
the folding line (L30) being set in the composite-member continuous body (30a),
in the folding step (S110),
the auxiliary-member continuous body (40a) is folded in the intersecting direction together with the composite-member continuous body (30a), and
in the second joining step (Sill),
the joining portion (ja, jb) is formed such that an overlapping portion is located in at least one of the intersecting-direction outer end portion (jaet, jbet) and the intersecting-direction inner end portion (jaei, jbei) of the joining portion (ja, jb),
the overlapping portion being a portion where portions of the auxiliary-member continuous body (40a) are overlapped due to the folding step (S110).

5. A method for manufacturing an absorbent article (1) according to claim 1 or 2, wherein
in the second joining step (Sill),
the auxiliary-member continuous body (40a) is arranged on both an intersecting-direction outer end portion (jaet, jbet) and an intersecting-direction inner end portion (jaei, jbei) of the joining portion (ja, jb) that is to be formed,
the intersecting-direction outer end portion (jaet, jbet) being an end portion located closer to outside in the intersecting direction,
the intersecting-direction inner end portion (jaei, jbei) being an end portion located closer to inside in the intersecting direction.

6. A method for manufacturing an absorbent article (1) according to any one of claims 1 to 3, wherein
the method further comprises a cutting step of cutting the auxiliary-member continuous body (40a) together with the composite-member continuous body (30a) at a cutting line,
the cutting step being performed after the second joining step (Sill),
the cutting line being a line that is set in the composite-member continuous body (30a) and extends in a curved shape along the direction of transport, and
the auxiliary-member continuous body (40a) is arranged at a portion that is constituted by the composite-member continuous body (30a) and that is in an edge portion of each of the leg openings (30HL) formed by the cutting step.

7. A method for manufacturing an absorbent article (1) according to claim 1 or 2, wherein
the method further comprises:
a leg-opening forming step (S109) of forming the leg openings (30HL) by performing cutting in a curved shape of the auxiliary-member continuous body (40a) and the composite-member continuous body (30a) so as to penetrate through the auxiliary-member continuous body (40a) and the composite-member continuous body (30a),
the auxiliary-member continuous body (40a) and the composite-member continuous body (30a) having been joined in the first joining step (S105); and
a folding step (S110) of folding the composite-member continuous body (30a) together with the auxiliary-member continuous body (40a) in the intersecting direction at a folding line (L30),
the folding step (S110) being performed after the leg-opening forming step (S109),
the folding line (L30) being set in the composite-member continuous body (30a), and
in the leg-opening forming step (S109), the leg openings (30HL) are each formed so as to straddle the folding line (L30) in the intersecting direction.

8. A method for manufacturing an absorbent article (1) according to any one of claims 1 to 7, wherein
in the second joining step (Sill),
a pair of the joining portions are formed in the absorbent-main-body continuous body (10a), on an upstream side in the direction of transport, and
a pair of the joining portions are formed in the absorbent-main-body continuous body (10a), on a downstream side in the direction of transport, and
letting the pair of joining portions on the upstream side be a pair of first joining portions (ja) and
letting the pair of joining portions on the downstream side be a pair of second joining portion (jb),
a plurality of weld portions are provided intermittently in the first joining portions (ja) and the second joining portions (jb),
the first joining portions (ja) are each provided so as to be inclined toward a center from outside in the intersecting direction, and
the second joining portions (jb) are each provided so as to be inclined toward outside in the intersecting direction from the center.

9. A method for manufacturing an absorbent article (1) according to any one of claims 1 to 8, wherein
the auxiliary member (40) has stretchability, and
an amount of elongation per unit length of the auxiliary-member continuous body (40a) in the dividing step (S103) is smaller than the amount of elongation per unit length of the auxiliary-member continuous body (40a) in the first joining step (S105).

10. A method for manufacturing an absorbent article (1) according to any one of claims 1 to 9, wherein
concerning a transporting distance of the auxiliary-member continuous body (40a) from dividing in the dividing step (S103) to joining in the second joining step (Sill), in manufacturing,
concerning a transporting distance of the auxiliary-member continuous body (40a) from feeding by the roll in the feeding step (S101) to the dividing in the dividing step (S103),
the former transporting distance is shorter than the latter transporting distance.

11. A method for manufacturing an absorbent article (1) according to any one of claims 1 to 10, wherein
the method further comprises a positioning step (S102) of performing positioning such that a central position of the auxiliary-member continuous body (40a) in the intersecting direction is at the predetermined position in the intersecting direction,
the positioning step being performed before the dividing step (S103).

12. A method for manufacturing an absorbent article (1) according to any one of claims 1 to 11, wherein
in the dividing step (S103),
the auxiliary-member continuous body (40a) is divided along a dividing line (L1),
the dividing line (L1) being a line that is continuous in the direction of transport, and
the method further comprises a positioning step (S104) of positioning the divided auxiliary-member continuous bodies based on divided ends with respect to the intersecting direction,
the positioning step being performed between the dividing step (S103) and the first joining step (S105).

13. A method for manufacturing an absorbent article (1) according to any one of claims 1 to 11, wherein
the dividing step (S103) is performed after the first joining step (S105'').

14. A method for manufacturing an absorbent article (1) according to claim 13, wherein
in the dividing step (S103),
the auxiliary-member continuous body (40a) is divided along a dividing line (L1),
the dividing line (L1) being a line that is continuous in the direction of transport, and
of the divided auxiliary-member continuous body (40a), ends (40aex) that have been divided in the intersecting direction, are arranged at ends of the leg openings (30HL).

15. A method for manufacturing an absorbent article (1) according to any one of claims 1 to 10, wherein
in the dividing step (S103),
the auxiliary-member continuous body (40a) is divided into at least four portions (40S1, 40S2, 40S3, 40S4) at a plurality of predetermined positions in the intersecting direction, and
in the first joining step (S105),
the at least four divided portions (40S1, 40S2, 40S3, 40S4) of the auxiliary-member continuous body (40a) are arranged such that, in a pair composed of the at least four divided portions, the two divided portions have an overlapping portion, and then are joined to the composite-member continuous body (30a).

## Patentansprüche

1. Verfahren zur Herstellung eines absorbierenden Artikels (1),
wobei der absorbierende Artikel (1) einen absorbierenden Hauptkörper (10), ein Paar von Verbundelementen (30) und ein Hilfselement (40) einschließt,
wobei der absorbierende Hauptkörper (10) zum Absorbieren einer Flüssigkeit vorgesehen ist,
das Paar von Verbundelementen (30) Beinöffnungen (30HL) und eine Taillenöffnung (1HB) aufweist,
das Hilfselement (40) auf jedem der Verbundelemente (30) bereitgestellt ist,
wobei das Verfahren Folgendes umfasst:
einen Zuführungsschritt (S101) des Zuführens eines kontinuierlichen Körpers des Hilfselements (40a) von einer Walze (61),
wobei der kontinuierliche Körper des Hilfselements (40a) zur Ausbildung des Hilfselements (40) vorgesehen ist,
der kontinuierliche Körper des Hilfselements (40a) um die Walze (61) gewunden ist;
einen ersten Verbindungsschritt (S105) des Verbindens eines kontinuierlichen Körpers des Verbundelements (30a) und des kontinuierlichen Körpers des Hilfselements (40a), der von der Walze zugeführt wurde,
wobei der kontinuierliche Körper des Verbundelements (30a) zur Ausbildung des Verbundelements (30) vorgesehen ist;
einen zweiten Verbindungsschritt (S111) des Verbindens eines kontinuierlichen Körpers des absorbierenden Hauptkörpers (10a) mit dem kontinuierlichen Körper des Hilfselements (40a) und dem kontinuierlichen Körper des Verbundelements (30a), die in dem ersten Verbindungsschritt (S105) verbunden wurden,
wobei der zweite Verbindungsschritt (S111) durch Ausbilden eines Verbindungsteils (ja, jb) durchgeführt wird,
der Verbindungsteil (ja, jb) eine Förderrichtung kreuzt,
der kontinuierliche Körper des absorbierenden Hauptkörpers (10a) zur Ausbildung des absorbierenden Hauptkörpers (10) vorgesehen ist; und
einen Teilungsschritt (S103) des Teilens des kontinuierlichen Körpers des Hilfselements (40a) an einer vorgegebenen Position in einer kreuzenden Richtung,
wobei der Teilungsschritt (S103) zwischen dem Zuführungsschritt (S101) und dem zweiten Verbindungsschritt (S111) durchgeführt wird,
die kreuzende Richtung eine Richtung ist, die die Förderrichtung kreuzt.

2. Verfahren zur Herstellung eines absorbierenden Artikels (1) nach Anspruch 1, wobei
das Hilfselement (40) ein dehnbares Element ist.

3. Verfahren zur Herstellung eines absorbierenden Artikels (1) nach Anspruch 1 oder 2, wobei
in dem zweiten Verbindungsschritt (S111)
der kontinuierliche Körper des Hilfselements (40a) derart angeordnet ist, dass er mindestens einen von einem äußeren Endteil in kreuzender Richtung (jaet, jbet) und einem inneren Endteil in kreuzender Richtung (jaei, jbei) des Verbindungsteils (ja, jb), das auszubilden ist, überlappt,
wobei der äußere Endteil in kreuzender Richtung (jaet, jbet) ein Endteil ist, der sich näher nach außen in der kreuzenden Richtung befindet,
der innere Endteil in kreuzender Richtung (jaei, jbei) ein Endteil ist, der sich näher nach innen in der kreuzenden Richtung befindet.

4. Verfahren zur Herstellung eines absorbierenden Artikels (1) nach Anspruch 3, wobei
das Verfahren weiter einen Faltungsschritt (S110) des Faltens des kontinuierlichen Körpers des Verbundelements (30a) in der kreuzenden Richtung an einer Faltlinie (L30) umfasst,
wobei der Faltungsschritt (S110) zwischen dem ersten Verbindungsschritt (S105) und dem zweiten Verbindungsschritt (S111) durchgeführt wird,
die Faltlinie (L30) in dem kontinuierlichen Körper des Verbundelements (30a) festgelegt ist,
wobei in dem Faltungsschritt (SIlO)
der kontinuierliche Körper des Hilfselements (40a) in der kreuzenden Richtung zusammen mit dem kontinuierlichen Körper des Verbundelements (30a) gefaltet wird und
wobei in dem zweiten Verbindungsschritt (S111)
der Verbindungsteil (ja, jb) derart ausgebildet ist, dass sich ein überlappender Teil in mindestens einem von dem äußeren Endteil in kreuzender Richtung (jaet, jbet) und dem inneren Endteil in kreuzender Richtung (jaei, jbei) des Verbindungsteils (ja, jb) befindet,
wobei der überlappende Teil ein Teil ist, in dem Teile des kontinuierlichen Körpers des Hilfselements (40a) aufgrund des Faltungsschritts (S110) überlappt sind.

5. Verfahren zur Herstellung eines absorbierenden Artikels (1) nach Anspruch 1 oder 2, wobei
in dem zweiten Verbindungsschritt (S111)
der kontinuierliche Körper des Hilfselements (40a) sowohl auf einem äußeren Endteil in kreuzender Richtung (jaet, jbet) als auch einem inneren Endteil in kreuzender Richtung (jaei, jbei) des Verbindungsteils (ja, jb), das auszubilden ist, angeordnet ist,
wobei der äußere Endteil in kreuzender Richtung (jaet, jbet) ein Endteil ist, der sich näher nach außen in der kreuzenden Richtung befindet,
der innere Endteil in kreuzender Richtung (jaei, jbei) ein Endteil ist, der sich näher nach innen in der kreuzenden Richtung befindet.

6. Verfahren zur Herstellung eines absorbierenden Artikels (1) nach einem der Ansprüche 1 bis 3, wobei
das Verfahren weiter einen Schneideschritt des Schneidens des kontinuierlichen Körpers des Hilfselements (40a) zusammen mit dem kontinuierlichen Körper des Verbundelements (30a) an einer Schnittlinie umfasst,
wobei der Schneideschritt nach dem zweiten Verbindungsschritt (S111) durchgeführt wird,
die Schnittlinie eine Linie ist, die in dem kontinuierlichen Körper des Verbundelements (30a) festgelegt ist und sich in einer gebogenen Form entlang der Förderrichtung erstreckt, und
wobei der kontinuierliche Körper des Hilfselements (40a) an einem Teil angeordnet ist, der von dem kontinuierlichen Körper des Verbundelements (30a) gebildet wird und der in einem Randteil von jeder der Beinöffnungen (30HL) vorliegt, die durch den Schneideschritt ausgebildet werden.

7. Verfahren zur Herstellung eines absorbierenden Artikels (1) nach Anspruch 1 oder 2, wobei
das Verfahren weiter Folgendes umfasst:
einen Bildungsschritt der Beinöffnung (S109) des Bildens der Beinöffnungen (30HL) durch Durchführen des Schneidens in einer gebogenen Form des kontinuierlichen Körpers des Hilfselements (40a) und des kontinuierlichen Körpers des Verbundelements (30a) derart, dass der kontinuierliche Körper des Hilfselements (40a) und der kontinuierliche Körper des Verbundelements (30a) durchdrungen werden,
wobei der kontinuierliche Körper des Hilfselements (40a) und der kontinuierliche Körper des Verbundelements (30a) in dem ersten Verbindungsschritt (S105) verbunden wurden; und
einen Faltungsschritt (S110) des Faltens des kontinuierlichen Körpers des Verbundelements (30a) zusammen mit dem kontinuierlichen Körper des Hilfselements (40a) in der kreuzenden Richtung an einer Faltlinie (L30),
wobei der Faltungsschritt (S110) nach dem Bildungsschritt der Beinöffnung (S109) durchgeführt wird,
die Faltlinie (L30) in dem kontinuierlichen Körper des Verbundelements (30a) festgelegt ist, und
wobei in dem Bildungsschritt der Beinöffnung (S109) die Beinöffnungen (30HL) jeweils derart gebildet werden, dass sie die Faltlinie (L30) in der kreuzenden Richtung überspannen.

8. Verfahren zur Herstellung eines absorbierenden Artikels (1) nach einem der Ansprüche 1 bis 7, wobei
in dem zweiten Verbindungsschritt (S111)
ein Paar der Verbindungsteile in dem kontinuierlichen Körper des absorbierenden Hauptkörpers (10a) auf einer vorgelagerten Seite in der Förderrichtung ausgebildet wird und
ein Paar der Verbindungsteile in dem kontinuierlichen Körper des absorbierenden Hauptkörpers (10a) auf einer nachgelagerten Seite in der Förderrichtung ausgebildet wird, und
wenn das Paar der Verbindungsteile auf der vorgelagerten Seite ein Paar von ersten Verbindungsteilen (ja) ist und
wenn das Paar von Verbindungsteilen auf der nachgelagerten Seite ein Paar von zweiten Verbindungsteilen (jb) ist,
eine Vielzahl von Schweißteilen intermittierend in den ersten Verbindungsteilen (ja) und den zweiten Verbindungsteilen (jb) bereitgestellt wird,
die ersten Verbindungsteile (ja) jeweils derart bereitgestellt werden, dass sie zu einer Mitte von außen in der kreuzenden Richtung geneigt sind, und
die zweiten Verbindungsteile (jb) jeweils derart bereitgestellt werden, dass sie nach außen in der kreuzenden Richtung von der Mitte geneigt sind.

9. Verfahren zur Herstellung eines absorbierenden Artikels (1) nach einem der Ansprüche 1 bis 8, wobei
das Hilfselement (40) eine Dehnbarkeit aufweist und
ein Maß der Dehnung pro Einheitslänge des kontinuierlichen Körpers des Hilfselements (40a) in dem Teilungsschritt (S103) kleiner ist als das Maß der Dehnung pro Einheitslänge des kontinuierlichen Körpers des Hilfselements (40a) in dem ersten Verbindungsschritt (S105).

10. Verfahren zur Herstellung eines absorbierenden Artikels (1) nach einem der Ansprüche 1 bis 9, wobei
bezüglich einer Förderstrecke des kontinuierlichen Körpers des Hilfselements (40a) vom Teilen in dem Teilungsschritt (S103) zum Verbinden in dem zweiten Verbindungsschritt (S111) beim Herstellen,
bezüglich einer Förderstrecke des kontinuierlichen Körpers des Hilfselements (40a) vom Zuführen durch die Walze in dem Zuführungsschritt (S101) zum Teilen in dem Teilungsschritt (S103)
die erstere Förderstrecke kürzer ist als die letztere Förderstrecke.

11. Verfahren zur Herstellung eines absorbierenden Artikels (1) nach einem der Ansprüche 1 bis 10, wobei
das Verfahren weiter einen Positionierungsschritt (S102) des Durchführens einer Positionierung derart umfasst, dass eine mittlere Position des kontinuierlichen Körpers des Hilfselements (40a) in der kreuzenden Richtung an der vorgegebenen Position in der kreuzenden Richtung vorliegt,
wobei der Positionierungsschritt vor dem Teilungsschritt (S103) durchgeführt wird.

12. Verfahren zur Herstellung eines absorbierenden Artikels (1) nach einem der Ansprüche 1 bis 11, wobei
in dem Teilungsschritt (S103)
der kontinuierliche Körper des Hilfselements (40a) entlang einer Teilungslinie (L1) geteilt wird,
wobei die Teilungslinie (L1) eine Linie ist, die in der Förderrichtung durchgehend ist, und
wobei das Verfahren weiter einen Positionierungsschritt (S104) des Positionierens der geteilten kontinuierlichen Körper des Hilfselements bezogen auf geteilte Enden hinsichtlich der kreuzenden Richtung umfasst,
wobei der Positionierungsschritt zwischen dem Teilungsschritt (S103) und dem ersten Verbindungsschritt (S105) durchgeführt wird.

13. Verfahren zur Herstellung eines absorbierenden Artikels (1) nach einem der Ansprüche 1 bis 11, wobei
der Teilungsschritt (S103) nach dem ersten Verbindungsschritt (S105") durchgeführt wird.

14. Verfahren zur Herstellung eines absorbierenden Artikels (1) nach Anspruch 13, wobei
in dem Teilungsschritt (S103)
der kontinuierliche Körper des Hilfselements (40a) entlang einer Teilungslinie (L1) geteilt wird,
wobei die Teilungslinie (L1) eine Linie ist, die in der Förderrichtung durchgehend ist, und
wobei Enden (40aex) des geteilten kontinuierlichen Körpers des Hilfselements (40a), die in der kreuzenden Richtung geteilt wurden, an Enden der Beinöffnungen (30HL) angeordnet werden.

15. Verfahren zur Herstellung eines absorbierenden Artikels (1) nach einem der Ansprüche 1 bis 10, wobei
in dem Teilungsschritt (S103)
der kontinuierliche Körper des Hilfselements (40a) in mindestens vier Teile (4051, 40S2, 40S3, 40S4) an einer Vielzahl von vorgegebenen Positionen in der kreuzenden Richtung geteilt wird und
in dem ersten Verbindungsschritt (S105)
die mindestens vier geteilten Teile (4051, 40S2, 40S3, 40S4) des kontinuierlichen Körpers des Hilfselements (40a) derart angeordnet werden, dass in einem Paar, das aus den mindestens vier geteilten Teilen besteht, die zwei geteilten Teile einen überlappenden Teil aufweisen und dann mit dem kontinuierlichen Körper des Verbundelements (30a) verbunden werden.

## Revendications

1. Procédé de fabrication d'un article absorbant (1),
l'article absorbant (1) comprenant un corps principal absorbant (10), une paire d'éléments composites (30), et un élément auxiliaire (40),
le corps principal absorbant (10) étant destiné à absorber un liquide,
la paire d'éléments composites (30) ayant des ouvertures pour les jambes (30HL) et une ouverture pour la taille (1HB),
l'élément auxiliaire (40) étant mis en oeuvre sur chacun des éléments composites (30),
le procédé comportant :
une étape d'acheminement (S101) consistant à acheminer un corps continu d'élément auxiliaire (40a) en provenance d'un rouleau (61),
le corps continu d'élément auxiliaire (40a) étant destiné à former l'élément auxiliaire (40),
le corps continu d'élément auxiliaire (40a) étant enroulé autour du rouleau (61) ;
une première étape d'assemblage (S105) consistant à assembler un corps continu d'élément composite (30a) et le corps continu d'élément auxiliaire (40a) qui a été acheminé en provenance du rouleau,
le corps continu d'élément composite (30a) étant destiné à former l'élément composite (30) ;
une deuxième étape d'assemblage (S111) consistant à assembler un corps continu de corps principal absorbant (10a) au niveau du corps continu d'élément auxiliaire (40a) et du corps continu d'élément composite (30a) qui ont été assemblés au cours de la première étape d'assemblage (S105),
la deuxième étape d'assemblage (S111) étant effectuée en formant une partie d'assemblage (ja, jb),
la partie d'assemblage (ja, jb) croisant une direction allant dans le sens du transport,
le corps continu de corps principal absorbant (10a) étant destiné à former le corps principal absorbant (10) ; et
une étape de division (S103) consistant à diviser le corps continu d'élément auxiliaire (40a) au niveau d'une position prédéterminée dans une direction allant dans le sens du croisement,
l'étape de division (S103) étant effectuée entre l'étape d'acheminement (S101) et la deuxième étape d'assemblage (S111),
la direction allant dans le sens du croisement étant une direction qui croise la direction allant dans le sens du transport.

2. Procédé de fabrication d'un article absorbant (1) selon la revendication 1, dans lequel
l'élément auxiliaire (40) est un élément étirable.

3. Procédé de fabrication d'un article absorbant (1) selon la revendication 1 ou la revendication 2, dans lequel
au cours de la deuxième étape d'assemblage (S111),
le corps continu d'élément auxiliaire (40a) est agencé de manière à chevaucher au moins l'une parmi une partie d'extrémité extérieure dans la direction allant dans le sens du croisement (jaet, jbet) et une partie d'extrémité intérieure dans la direction allant dans le sens du croisement (jaei, jbei) de la partie d'assemblage (ja, jb) qui est destinée à être formée,
la partie d'extrémité extérieure dans la direction allant dans le sens du croisement (jaet, jbet) étant une partie d'extrémité se trouvant plus près de l'extérieur dans la direction allant dans le sens du croisement,
la partie d'extrémité intérieure dans la direction allant dans le sens du croisement (jaei, jbei) étant une partie d'extrémité se trouvant plus près de l'intérieur dans la direction allant dans le sens du croisement.

4. Procédé de fabrication d'un article absorbant (1) selon la revendication 3, dans lequel
le procédé comporte par ailleurs une étape de pliage (S110) consistant à plier le corps continu d'élément composite (30a) dans la direction allant dans le sens du croisement au niveau d'une ligne de pliage (L30),
l'étape de pliage (S110) étant effectuée entre la première étape d'assemblage (S105) et la deuxième étape d'assemblage (S111),
la ligne de pliage (L30) se trouvant dans le corps continu d'élément composite (30a),
au cours de l'étape de pliage (S110),
le corps continu d'élément auxiliaire (40a) est plié dans la direction allant dans le sens du croisement en même temps que le corps continu d'élément composite (30a), et
au cours de la deuxième étape d'assemblage (S111),
la partie d'assemblage (ja, jb) est formée de telle sorte qu'une partie de chevauchement est située dans au moins l'une parmi la partie d'extrémité extérieure dans la direction allant dans le sens du croisement (jaet, jbet) et la partie d'extrémité intérieure dans la direction allant dans le sens du croisement (jaei, jbei) de la partie d'assemblage (ja, jb),
la partie de chevauchement étant une partie où des parties du corps continu d'élément auxiliaire (40a) sont chevauchées en raison de l'étape de pliage (S110).

5. Procédé de fabrication d'un article absorbant (1) selon la revendication 1 ou la revendication 2, dans lequel
au cours de la deuxième étape d'assemblage (S111),
le corps continu d'élément auxiliaire (40a) est agencé sur à la fois une partie d'extrémité extérieure dans la direction allant dans le sens du croisement (jaet, jbet) et une partie d'extrémité intérieure dans la direction allant dans le sens du croisement (jaei, jbei) de la partie d'assemblage (ja, jb) qui est destinée à être formée,
la partie d'extrémité extérieure dans la direction allant dans le sens du croisement (jaet, jbet) étant une partie d'extrémité se trouvant plus près de l'extérieur dans la direction allant dans le sens du croisement,
la partie d'extrémité intérieure dans la direction allant dans le sens du croisement (jaei, jbei) étant une partie d'extrémité se trouvant plus près de l'intérieur dans la direction allant dans le sens du croisement.

6. Procédé de fabrication d'un article absorbant (1) selon l'une quelconque des revendications 1 à 3, dans lequel
le procédé comporte par ailleurs une étape de découpe consistant à découper le corps continu d'élément auxiliaire (40a) en même temps que le corps continu d'élément composite (30a) au niveau d'une ligne de découpe,
l'étape de découpe étant effectuée après la deuxième étape d'assemblage (S111),
la ligne de découpe étant une ligne qui se trouve dans le corps continu d'élément composite (30a) et qui s'étend en une forme courbe le long de la direction allant dans le sens du transport, et
le corps continu d'élément auxiliaire (40a) est agencé au niveau d'une partie qui est constituée par le corps continu d'élément composite (30a) et qui est dans une partie de bord de chacune des ouvertures pour les jambes (30HL) formées par l'étape de découpe.

7. Procédé de fabrication d'un article absorbant (1) selon la revendication 1 ou la revendication 2, dans lequel
le procédé comporte par ailleurs :
une étape de formation d'ouvertures pour les jambes (S109) consistant à former les ouvertures pour les jambes (30HL) en effectuant une découpe en une forme courbe du corps continu d'élément auxiliaire (40a) et du corps continu d'élément composite (30a) de manière à pénétrer dans le corps continu d'élément auxiliaire (40a) et le corps continu d'élément composite (30a),
le corps continu d'élément auxiliaire (40a) et le corps continu d'élément composite (30a) ayant été assemblés au cours de la première étape d'assemblage (S105) ; et
une étape de pliage (S110) consistant à plier le corps continu d'élément composite (30a) en même temps que le corps continu d'élément auxiliaire (40a) dans la direction allant dans le sens du croisement au niveau d'une ligne de pliage (L30),
l'étape de pliage (S110) étant effectuée après l'étape de formation d'ouvertures pour les jambes (S109),
la ligne de pliage (L30) se trouvant dans le corps continu d'élément composite (30a), et
au cours de l'étape de formation d'ouvertures pour les jambes (S109), les ouvertures pour les jambes (30HL) sont chacune formées de manière à enjamber la ligne de pliage (L30) dans la direction allant dans le sens du croisement.

8. Procédé de fabrication d'un article absorbant (1) selon l'une quelconque des revendications 1 à 7, dans lequel
au cours de la deuxième étape d'assemblage (S111),
une paire des parties d'assemblage sont formées dans le corps continu de corps principal absorbant (10a), sur un côté en amont dans la direction allant dans le sens du transport, et
une paire des parties d'assemblage sont formées dans le corps continu de corps principal absorbant (10a), sur un côté en aval dans la direction allant dans le sens du transport, et
laisser la paire de parties d'assemblage sur le côté en amont être une paire de premières parties d'assemblage (ja) et
laisser la paire de parties d'assemblage sur le côté en aval être une paire de deuxièmes parties d'assemblage (jb),
une pluralité de parties de soudure sont mises en oeuvre de manière intermittente dans les premières parties d'assemblage (ja) et les deuxièmes parties d'assemblage (jb),
les premières parties d'assemblage (ja) sont chacune mises en oeuvre de manière à être inclinées vers un centre depuis l'extérieur dans la direction allant dans le sens du croisement, et
les deuxièmes parties d'assemblage (jb) sont chacune mises en oeuvre de manière à être inclinées vers l'extérieur dans la direction allant dans le sens du croisement depuis le centre.

9. Procédé de fabrication d'un article absorbant (1) selon l'une quelconque des revendications 1 à 8, dans lequel
l'élément auxiliaire (40) présente une aptitude à l'étirement, et
une quantité d'allongement par unité de longueur du corps continu d'élément auxiliaire (40a) au cours de l'étape de division (S103) est inférieure à la quantité d'allongement par unité de longueur du corps continu d'élément auxiliaire (40a) au cours de la première étape d'assemblage (S105).

10. Procédé de fabrication d'un article absorbant (1) selon l'une quelconque des revendications 1 à 9, dans lequel
en ce qui concerne une distance de transport du corps continu d'élément auxiliaire (40a) depuis la division au cours de l'étape de division (S103) jusqu'à l'assemblage au cours de la deuxième étape d'assemblage (S111) au cours de la fabrication,
en ce qui concerne une distance de transport du corps continu d'élément auxiliaire (40a) allant de l'acheminement par le rouleau au cours de l'étape d'acheminement (S101) jusqu'à la division au cours de l'étape de division (S103),
la première distance de transport est plus courte que la deuxième distance de transport.

11. Procédé de fabrication d'un article absorbant (1) selon l'une quelconque des revendications 1 à 10, dans lequel
le procédé comporte par ailleurs une étape de positionnement (S102) consistant à effectuer un positionnement de telle sorte qu'une position centrale du corps continu d'élément auxiliaire (40a) dans la direction allant dans le sens du croisement se trouve au niveau de la position prédéterminée dans la direction allant dans le sens du croisement,
l'étape de positionnement étant effectuée avant l'étape de division (S103).

12. Procédé de fabrication d'un article absorbant (1) selon l'une quelconque des revendications 1 à 11, dans lequel
au cours de l'étape de division (S103),
le corps continu d'élément auxiliaire (40a) est divisé le long d'une ligne de division (L1),
la ligne de division (L1) étant une ligne qui est continue dans la direction allant dans le sens du transport, et
le procédé comporte par ailleurs une étape de positionnement (S104) consistant à positionner les corps continus d'élément auxiliaire divisés en se basant sur des extrémités divisées par rapport à la direction allant dans le sens du croisement,
l'étape de positionnement étant effectuée entre l'étape de division (S103) et la première étape d'assemblage (S105).

13. Procédé de fabrication d'un article absorbant (1) selon l'une quelconque des revendications 1 à 11, dans lequel
l'étape de division (S103) est effectuée après la première étape d'assemblage (S105").

14. Procédé de fabrication d'un article absorbant (1) selon la revendication 13, dans lequel
au cours de l'étape de division (S103),
le corps continu d'élément auxiliaire (40a) est divisé le long d'une ligne de division (L1),
la ligne de division (L1) étant une ligne qui est continue dans la direction allant dans le sens du transport, et
en provenance du corps continu d'élément auxiliaire divisé (40a), des extrémités (40aex) qui ont été divisées dans la direction allant dans le sens du croisement, sont agencées au niveau d'extrémités des ouvertures pour les jambes (30HL).

15. Procédé de fabrication d'un article absorbant (1) selon l'une quelconque des revendications 1 à 10, dans lequel
au cours de l'étape de division (S103),
le corps continu d'élément auxiliaire (40a) est divisé en au moins quatre parties (40S1, 40S2, 40S3, 40S4) au niveau d'une pluralité de positions prédéterminées dans la direction allant dans le sens du croisement, et
au cours de la première étape d'assemblage (S105),
lesdites au moins quatre parties divisées (40S1, 40S2, 40S3, 40S4) du corps continu d'élément auxiliaire (40a) sont agencées de telle sorte que, dans une paire composée desdites au moins quatre partie divisées, les deux parties divisées ont une partie de chevauchement, et puis elles sont assemblées sur le corps continu d'élément composite (30a).
